(19) **Europäisches Patentamt / European Patent Office / Office européen des brevets**

(11) **EP 4 726 051 A1**

(12) **EUROPEAN PATENT APPLICATION**
published in accordance with Art. 153(4) EPC

(43) Date of publication:
**15.04.2026 Bulletin 2026/16**

(21) Application number: **24819350.0**

(22) Date of filing: **05.06.2024**

(51) International Patent Classification (IPC):
**C12P 21/00** *(2006.01)*  **C12N 1/19** *(2006.01)*
**C12N 15/81** *(2006.01)*

(52) Cooperative Patent Classification (CPC):
**C12N 15/81; C12P 21/00**

(86) International application number:
**PCT/JP2024/020485**

(87) International publication number:
**WO 2024/253122 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.06.2023 JP 2023092875**

(71) Applicant: **FUJIFILM Corporation**
**Tokyo 106-8620 (JP)**

(72) Inventors:
• **DE BOER, Arjo Lysander**
  **5047 TK Tilburg (NL)**
• **SAKAI, Hidekazu**
  **Kanagawa 258-8577 (JP)**
• **FUJIWARA, Sayami**
  **Kanagawa 258-8577 (JP)**
• **FUKUNAGA, Kazuto**
  **Kanagawa 258-8577 (JP)**
• **HIRATSUKA, Takahiro**
  **Kanagawa 258-8577 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **RECOMBINANT YEAST, METHOD FOR PRODUCING TARGET PROTEIN, AND KIT AND METHOD FOR PRODUCING RECOMBINANT YEAST**

(57) An object of the present invention is to provide a recombinant yeast that can highly express a target protein in a tightly regulated manner without using methanol, and to provide a method for producing a target protein using the recombinant yeast, a kit for producing the recombinant yeast, and a method for producing the recombinant yeast. According to the present invention, there is provided a recombinant yeast containing a first exogenous sucrose-inducible promoter for expressing a target protein.

EP 4 726 051 A1

## Description

### Technical Field

The present invention relates to a recombinant yeast for expressing a target protein. The present invention further relates to a method for producing a target protein using the recombinant yeast, a kit for producing the recombinant yeast, and a method for producing the recombinant yeast.

### Background Art

Recombinant proteins are useful as a pharmaceutical or an industrial enzyme, and are produced by a recombinant microorganism in many cases. Methanol-assimilating yeasts have been utilized as an efficient protein expression system. As methanol-assimilating yeasts that enables high expression of a protein and high-density culture, Ogataea polymorpha, Candida boidinii, Ogataea methanolica, and Pichia pastoris (Komagataella phaffii) are known. In particular, Pichia pastoris has been extensively utilized in commercial applications.

As a promoter for expressing a protein in Pichia pastoris, an AOX1 promoter has been utilized. The AOX1 promoter is a tightly regulated methanol-inducible promoter, and enables strong inducible expression using methanol as a carbon source.

Furthermore, in recent years, as an alternative promoter that does not use methanol, an AOX1 promoter mutant (Patent Document 1), a G1 promoter mutant (Patent Document 2), an FMD/MOX promoter (Patent Document 3), and the like have been developed.

### Prior Art Documents

### Patent Documents

Patent Document 1: WO2006/089329A
Patent Document 2: WO2017/021541A
Patent Document 3: WO2017/109082A

### Summary of Invention

### Object to be solved by the invention

In the use of the AOX1 promoter, methanol is used. However, in a case of using methanol which is flammable and toxic, it is necessary to take explosion-proof measures and to address health risks. Therefore, there is a problem that expensive equipment is required in particular in a case of industrial use on a large scale. In addition, by using methanol as a carbon source, the survival rate of the cell is reduced as compared with a carbon source such as glucose, which may affect the yield of the product. Therefore, even in a case where an increase in protein expression is desired, the amount of the carbon source to be added is limited. Furthermore, heat generation due to oxygen consumption and metabolism is large, and the increase in cooling cost is also a problem.

On the other hand, the promoters described in WO2006/089329A, WO2017/021541A, and WO2017/109082A have a problem that the expression ability is lower than or equal to that of the AOX1 promoter, or it is difficult to achieve tight regulation of expression because of the expression under repression-release conditions. In addition, in a case of expressing various exogenous genes to improve the productivity of the protein (for example, in a case where protein A is expressed to function as a chaperone for target protein B), a plurality of types of alternative promoters are required, but there is a limitation in the number in a case of using existing promoters.

Therefore, a novel promoter that does not use methanol, tightly regulates expression, and has a high expression level is required.

An object to be achieved by the present invention is to provide a recombinant yeast that can highly express a target protein in a tightly regulated manner without using methanol. Another object to be achieved by the present invention is to provide a method for producing a target protein using the recombinant yeast, a kit for producing the recombinant yeast, and a method for producing the recombinant yeast.

## Means for solving the object

As a result of intensive studies to achieve the above object, the present inventors have found that, by using an exogenous sucrose-inducible promoter as a promoter for expressing a target protein in a yeast, the target protein can be highly expressed in a tightly regulated manner without using methanol. The present invention has been completed based on the above findings.

According to an aspect of the present invention, the following invention is provided.

<1> A recombinant yeast comprising a first exogenous sucrose-inducible promoter for expressing a target protein.

<2> The recombinant yeast according to <1>, further comprising an exogenous gene encoding the target protein.

<3> The recombinant yeast according to <1> or <2>, further comprising an exogenous gene that confers sucrose assimilation.

<4> The recombinant yeast according to <3>, in which the exogenous gene that confers sucrose assimilation is linked to a second exogenous sucrose-inducible promoter.

<5> The recombinant yeast according to <3> or <4>, in which the exogenous gene that confers sucrose assimilation is a maltase gene.

<6> The recombinant yeast according to any one of <3> to <5>, in which the first exogenous sucrose-inducible promoter and the exogenous gene that confers sucrose assimilation are derived from a yeast having an $\alpha$-glucosidase which has sucrose-hydrolyzing activity.

<7> The recombinant yeast according to <6>, in which the yeast having an $\alpha$-glucosidase which has sucrose-hydrolyzing activity belongs to any of the genus Ogataea, Candida, Cyberlindnera, Wickerhamomyces, Clavispora, Debaryomyces, Meyerozyma, Scheffersomyces, Metschnikowia, Spathaspora, Babjeviella, Hypopichia, or Yamadazyma.

<8> The recombinant yeast according to <7>, in which the yeast having an $\alpha$-glucosidase which has sucrose-hydrolyzing activity is Ogataea parapolymorpha.

<9> The recombinant yeast according to any one of <1> to <8>, in which the recombinant yeast is the genus Komagataella.

<10> A method for producing a target protein, comprising culturing the recombinant yeast according to any one of <1> to <9> in a culture medium containing sucrose as a carbon source.

<11> The method for producing a target protein according to <10>, in which the target protein is an antibody, a bioactive protein, a bioactive polypeptide, an extracellular matrix, an artificial protein, or an enzyme.

<12> The method for producing a target protein according to <10> or <11>, in which a concentration of the sucrose in the culture medium during the culture is 0.5 to 2 g/mL.

<13> The method for producing a target protein according to any one of <10> to <12>, in which an addition rate of the sucrose is 2 to 100 g/hour per 0.9 L of the culture medium at the start of the culture.

<14> A kit for producing the recombinant yeast according to any one of <1> to <9>, the kit comprising a yeast and an expression construct containing a sucrose-inducible promoter.

<15> The kit according to <14>, in which the expression construct contains an exogenous gene encoding the target protein, and the sucrose-inducible promoter induces expression of the exogenous gene encoding the target protein.

<16> A method for producing a recombinant yeast, which is a method for producing the recombinant yeast according to any one of <1> to <9>, the method comprising introducing an expression construct containing a sucrose-inducible promoter into a yeast.

<17> The method for producing a recombinant yeast according to <16>, in which the expression construct containing the sucrose-inducible promoter is introduced into a genome of the yeast.

<18> The method for producing a recombinant yeast according to <16> or <17>, in which the expression construct containing the sucrose-inducible promoter contains an exogenous gene encoding the sucrose-inducible promoter and the target protein, and the sucrose-inducible promoter is an expression construct that induces expression of an exogenous gene encoding the target protein.

<19> The method for producing a recombinant yeast according to <16>, in which the expression construct containing the sucrose-inducible promoter is introduced into a genome of a yeast having an exogenous gene that confers sucrose assimilation.

## Effect of the invention

According to the present invention, the target protein can be highly expressed in a tightly regulated manner without using methanol.

**BRIEF DESCRIPTION OF THE DRAWINGS**

[FIG. 1] FIG. 1 shows a MAL cluster region for converting an AOX2 CDS of Pichia pastoris. The cluster region includes AG1, AGT1, and MAL-activator, and has sucrose assimilation.
[FIG. 2] FIG. 2 shows a relationship between product A and product B.
[FIG. 3] FIG. 3 shows screening of a MAL cluster-introduced yeast strain using a YNB-sucrose-supplemented medium. Left: Colony formation of the MAL cluster-introduced yeast strain was observed. Right: No colony was formed in the MAL cluster non-introduced yeast strain.
[FIG. 4] FIG. 4 shows a construction of a plasmid vector having the produced sucrose-inducible promoter.
[FIG. 5] FIG. 5 is a schematic diagram showing that a linearized plasmid is inserted into a MAL1 site on the MAL cluster by homologous recombination.
[FIG. 6] FIG. 6 shows a construction of a plasmid vector having the produced methanol-inducible promoter.

**Embodiments for carrying out the invention**

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" represents a range including numerical values described before and after "to" as a minimum value and a maximum value.

<Recombinant yeast>

The recombinant yeast according to the embodiment of the present invention contains a first exogenous sucrose-inducible promoter for expressing a target protein.

The first exogenous sucrose-inducible promoter in the present invention is a promoter for expressing a target protein. The first exogenous sucrose-inducible promoter is located upstream of a gene encoding a target protein to induce expression of the gene encoding the target protein. By using the sucrose-inducible promoter, the target protein can be expressed in a tightly regulated manner.

The gene encoding the target protein may be a gene originally present in the yeast or an exogenous gene, but is preferably the exogenous gene. That is, the recombinant yeast according to the embodiment of the present invention preferably further contains an exogenous gene encoding a target protein.

The first exogenous sucrose-inducible promoter is not particularly limited, and for example, a promoter of an $\alpha$-glucosidase gene, an $\alpha$-glucoside permease gene, or a MAL-activator gene contained in a MAL cluster described in Katrin Viigand et al., Genome Mining of Non-Conventional Yeasts: Search and Analysis of MAL Clusters and Proteins, Genes 2018, 9, 354; doi:10.3390/genes9070354 can be used. Details of the $\alpha$-glucosidase gene, the $\alpha$-glucoside permease gene, or the MAL-activator gene will be described later.

The first exogenous sucrose-inducible promoter is more preferably a promoter of an $\alpha$-glucosidase gene and still more preferably a promoter of an AG1 gene among the above. Specific examples thereof include a promoter having a nucleotide sequence set forth in SEQ ID NO: 16.

The promoter of the AG1 gene may be a promoter having a nucleotide sequence having a sequence identity of 80% or more, preferably 85% or more, more preferably 90% or more, still more preferably 95% or more, and particularly preferably 98% or more with the nucleotide sequence set forth in SEQ ID NO: 16 as long as it has promoter activity.

The sequence identity refers to a value calculated by the following equation.

$$\% \text{ Sequence identity} = [(\text{number of identical bases})/(\text{alignment length})] \times 100$$

The sequence identity between two nucleotide sequences can be determined by any method known to those skilled in the art, and can be determined using the Basic Local Alignment Search Tool (BLAST) program (J. Mol. Biol. 215: 403-410, 1990) or the like.

The recombinant yeast according to the embodiment of the present invention preferably further includes an exogenous gene that confers sucrose assimilation.

As the exogenous gene that confers sucrose assimilation, a gene encoding an enzyme involved in sucrose assimilation, a transporter gene involved in sucrose conversion, a gene encoding a transcriptional activator of the gene encoding an enzyme involved in the sucrose assimilation, or the like can be used.

Specific examples of the exogenous gene that confers sucrose assimilation include an $\alpha$-glucosidase gene, an

α-glucoside permease gene, a MAL-activator gene (such as those described in Genes 2018, 9, 354; doi:10.3390/genes9070354), and the like.

Examples of the α-glucosidase gene include a maltase gene, an isomaltase gene, a sucrase gene and the like. Specific examples of the maltase gene (including a gene having a function of the isomaltase gene in part) include MAL32 (S. cerevisiae), MAL1 (O. polymorpha), AG1 (O. parapolymorpha), AG1 (L. elongisporus), AG2 (M. guiliermondii), AG1.2 (C. fabianii), AGL1 (S. stipitis), MAL6 (S. stipitis), MAL7 (S. stipitis), MAL8 (S. stipitis), MalT (A. oryzae), AG1 (L. starkeyi), AG2 (L. starkeyi), AG4 (L. starkeyi), AG5 (L. starkeyi), AG6 (L. starkeyi), and examples of the isomaltase gene include AG1 (T. delbrueckii), AG1 (M. guiliermondii), AG1.1 (C. fabianii), AG3 (L. starkeyi), AG7 (L. starkeyi), AG8 (L. starkeyi), and the like.

Examples of the α-glucoside permease gene include MAL1 (S. stipitis), MAL2 (O. polymorpha), MAL2 (S. stipitis), MAL3 (S. stipitis), MAL4 (S. stipitis), MAL5 (S. stipitis), MAL31 (S. cerevisiae), MalP (A. oryzae), AGT1 (O. parapolymorpha), AGT1.1 (M. guiliermondii), AGT1.2 (M. guiliermondii), AGT1.3 (M. guiliermondii), AGT2.1 (L. starkeyi), AGT2.2 (L. starkeyi), AGT3 (L. starkeyi), AGT4 (L. starkeyi), AGT5 (L. starkeyi), AGT6 (L. starkeyi), and the like.

Examples of the MAL-activator gene include MAL33 (S. cerevisiae), MAL-ACT (O. parapolymorpha), MAL-ACT1 (O. polymorpha), MAL-ACT2 (O. polymorpha), MalR (A. oryzae), SUC1.1 (S. stipitis), SUC1.2 (S. stipitis), SUC1.4 (S. stipitis), and the like.

It is preferable that the exogenous gene that confers sucrose assimilation is linked to the second exogenous sucrose-inducible promoter.

The second exogenous sucrose-inducible promoter may be the same promoter as the first exogenous sucrose-inducible promoter or may be a different promoter from the first exogenous sucrose-inducible promoter, but it is preferably the same promoter as the first exogenous sucrose-inducible promoter.

Specific examples of the second exogenous sucrose-inducible promoter include the same examples as those described for the first exogenous sucrose-inducible promoter.

The first sucrose-inducible promoter and the exogenous gene that confers sucrose assimilation are preferably derived from a yeast having an α-glucosidase which has sucrose-hydrolyzing activity.

Examples of the yeast having an α-glucosidase which has sucrose-hydrolyzing activity include a yeast belonging to any of the genus Ogataea, Candida, Cyberlindnera, Wickerhamomyces, Clavispora, Debaryomyces, Meyerozyma, Scheffersomyces, Metschnikowia, Spathaspora, Babjeviella, Hypopichia, or Yamadazyma, but the yeast is not particularly limited. The yeast having an α-glucosidase which has sucrose-hydrolyzing activity is more preferably the genus Ogataea and particularly preferably Ogataea parapolymorpha.

In a case where the recombinant yeast according to the embodiment of the present invention has the exogenous gene that confers sucrose assimilation, a positional relationship between the gene encoding the target protein and the exogenous gene that confers sucrose assimilation is not particularly limited. The gene encoding the target protein may be inserted into the genome of the recombinant yeast or may be present outside the chromosome. The exogenous gene that confers sucrose assimilation may also be inserted into the genome of the recombinant yeast or may be present outside the chromosome. In a case where the gene encoding the target protein and the exogenous gene that confers sucrose assimilation are inserted into the genome of the recombinant yeast, insertion positions of the genes on the genome are not particularly limited. The gene encoding the target protein and the exogenous gene that confers sucrose assimilation may be inserted at distant positions on the genome or may be present at adjacent positions to each other. In a case where the gene encoding the target protein and the exogenous gene that confers sucrose assimilation are present at adjacent positions to each other, the gene encoding the target protein may be present upstream or downstream of the exogenous gene that confers sucrose assimilation.

Examples of the recombinant yeast according to the embodiment of the present invention include a yeast belonging to Komagataella, Pichia, Ogataea, Candida, Saccharomyces, Torulopsis, Zygosaccharomyces, Schizosaccharomyces, Yarrowia, Kluyveromyces, Debaryomyces, Geotrichum, Wickerhamomyces, Fellomyces, Sporobolomyces, and the like.

In the genus Komagataella, Komagataella pastoris, Komagataella phaffii, or the like is preferable. Both Komagataella pastoris and Komagataella phaffii have the synonym Pichia pastoris.

In the genus Pichia, Pichia methanolica or the like is preferable. In the genus Ogataea, Ogataea angusta, Ogataea polymorpha, Ogataea parapolymorpha, Ogataea minuta, or the like is preferable. In the genus Candida, Candida boidinii or the like is preferable.

Among the above, the recombinant yeast according to the embodiment of the present invention is particularly preferably the genus Komagataella. Specific examples of the strain that can be used include Komagataella pastoris ATCC 76273 (Y-11430, Pichia pastoris CBS7435). This strain can be obtained from American Type Culture Collection, Thermo Fisher Scientific, Inc., or the like.

<Kit for producing recombinant yeast>

The kit for producing the recombinant yeast according to the embodiment of the present invention includes a yeast and an expression construct containing a sucrose-inducible promoter.

As the yeast, the yeast belonging to the above-described genus for the recombinant yeast can be used, but the yeast is not particularly limited. The yeast is particularly preferably the genus Komagataella.

The expression construct preferably contains an exogenous gene encoding a target protein, and the above-described sucrose-inducible promoter induces expression of the exogenous gene encoding the target protein.

The expression construct containing the sucrose-inducible promoter can be obtained by inserting the sucrose-inducible promoter into an appropriate vector. The vector for inserting the sucrose-inducible promoter is not particularly limited as long as it is replicable in the host, and examples thereof include a plasmid, a phage DNA, a virus vector, and the like. In addition, in a case where the vector itself does not have a replication ability, a DNA fragment that can be replicated by being inserted into a chromosome of the host may be used.

Examples of the plasmid include a plasmid derived from Escherichia coli (for example, pBR322, pBR325, pUC118, pUC119, pUC18, pUC19, pBluescript, and the like), a plasmid derived from Bacillus subtilis (for example, pUB110, pTP5, and the like), a plasmid derived from yeast (for example, a YEp series such as YEp13, a YRp series, a Yip series, a YCp system such as YCp50, and the like, a Pichia vector system such as pPIC9, pPICZ, and pPICZα), and the like. Examples of the phage DNA include λ phage (Charon 4A, Charon 21A, EMBL3, EMBL4, λgt10, λgt11, λZAP, and the like). Furthermore, an animal virus such as a retrovirus or a vaccinia virus, or an insect virus vector such as a baculovirus can also be used.

To insert the sucrose-inducible promoter into the vector, first, the purified DNA may be cut with an appropriate restriction enzyme and then inserted into a restriction enzyme site or a multi-cloning site of an appropriate vector DNA to be linked to the vector. Alternatively, the vector and the sucrose-inducible promoter may be linked to each other by an in vitro method using PCR or the like or an in vivo method using yeast or the like by providing a region having homology to each other in a part of the vector and a part of the sucrose-inducible promoter.

It is preferable that the expression construct containing the sucrose-inducible promoter further contains an exogenous gene encoding a target protein downstream of the sucrose-inducible promoter. The method of inserting the exogenous gene is the same as the method of inserting the sucrose-inducible promoter into the vector.

<Method for producing recombinant yeast>

The method for producing the recombinant yeast according to the embodiment of the present invention includes introducing an expression construct containing a sucrose-inducible promoter into a yeast.

As the expression construct containing the sucrose-inducible promoter, and the yeast, those described above in the present specification can be used. As the expression construct containing the sucrose-inducible promoter, it is preferable that the expression construct contains the sucrose-inducible promoter and an exogenous gene encoding a target protein, and the sucrose-inducible promoter induces expression of the exogenous gene encoding the target protein.

The method of introducing the expression construct containing the sucrose-inducible promoter into the yeast is not particularly limited as long as it is a method of introducing DNA into the yeast, and examples thereof include electroporation, spheroplast method, lithium acetate method, and the like.

The expression construct containing the sucrose-inducible promoter may be introduced into the genome of the yeast, or the expression construct containing the sucrose-inducible promoter may be introduced into the yeast as extrachromosomal DNA, but is preferably introduced into the genome of the yeast. The introduction into the genome of the yeast may be performed by homologous recombination or may be performed in an aspect other than homologous recombination.

In a preferred aspect of the present invention, the expression construct containing the sucrose-inducible promoter is introduced into the yeast having the exogenous gene that confers sucrose assimilation. Preferably, the expression construct containing the sucrose-inducible promoter is introduced into the genome of the yeast having the exogenous gene that confers sucrose assimilation.

The presence or absence of the incorporation of the sucrose-inducible promoter into the yeast can be confirmed by a polymerase chain reaction (PCR) method, a Southern hybridization method, or the like. For example, DNA is prepared from the transformant, a DNA-specific primer is designed, and PCR is performed. Thereafter, the amplification product is subjected to agarose gel electrophoresis, polyacrylamide gel electrophoresis, capillary electrophoresis, or the like, is stained with ethidium bromide or the like, and is detected as one band, whereby it can be confirmed that the transformation has been performed.

In the recombinant yeast according to the embodiment of the present invention, selection can be performed by introducing an antibiotic resistance gene as a selection marker in advance, culturing the yeast using a culture medium containing the antibiotic, and acquiring a colony that has proliferated. As the antibiotic, zeocin, kanamycin, hygromycin, puromycin, blasticidin, or the like can be used.

The recombinant yeast into which the expression construct containing the sucrose-inducible promoter is introduced, which is produced as described above, can be cultured and grown by an appropriate conventional method and then used in the method for producing a target protein.

<Method for producing target protein>

The method for producing a target protein according to the embodiment of the present invention includes culturing the recombinant yeast according to the embodiment of the present invention in a culture medium containing sucrose as a carbon source.

As the sucrose source, biomass, a chemically synthesized product, or the like can be used, but the sucrose source is not particularly limited.

A concentration of the sucrose in the culture medium during the culture is preferably 0.5 to 2 g/mL, more preferably 0.5 to 0.7 g/mL, and still more preferably 0.55 to 0.65 g/mL.

A addition rate of the sucrose is preferably 2 to 100 g/hour, more preferably 2 to 20 g/hour, and still more preferably 3 to 15 g/hour per 0.9 L of the culture medium at the start of the culture.

Examples of the target protein include an antibody, a bioactive protein, a bioactive polypeptide, an extracellular matrix, an artificial protein, an enzyme, and the like, but the target protein is not particularly limited.

The antibody is a heterotetrameric protein in which two polypeptide chains of L chains and two polypeptide chains of H chains are linked by a disulfide bond, and is not particularly limited as long as it has an ability to bind to a specific antigen. The antibody may be a partial antibody (fragmented antibody). Examples of the partial antibody include an Fab antibody, an (Fab)2 antibody, an scFv antibody, a diabody, and the like, but the partial antibody is not particularly limited. The antibody is preferably a human antibody, a humanized antibody, a chimeric antibody, a mouse antibody, a bispecific antibody, or the like. The class of the antibody is also not particularly limited, and may be any one class of IgG such as IgG1, IgG2, IgG3, and IgG4, IgA, IgD, IgE, or IgM, but in a case of being used as a medicine, IgG and IgM are preferable.

Examples of the bioactive protein and the bioactive polypeptide include a hormone, a growth factor, a coagulation factor, a neuroprotein, an apolipoprotein, a tumor suppressor, an antigen including an antigenic protein and peptide of an endogenous or exogenous tumor, a bacterial surface protein and peptide, a viral surface protein and peptide, a protozoan cell surface protein and peptide, a fungal surface protein and peptide, and a parasitic substance including a viral reverse transcriptase and a related viral specific enzyme. Examples of the other proteins include a receptor such as an insulin receptor, a hormone receptor, and a growth factor receptor. In Examples described later, recombinant gelatin was used as the target protein.

Examples of the enzyme include an enzyme derived from a microorganism and an enzyme produced by an animal or a plant. Examples of the enzyme include phytase, amylase, glucosidase, cellulase, lipase, protease, glutaminase, peptidase, nuclease, oxidase, lactase, xylanase, trypsin, pectinase, isomerase, and the like, but the enzyme is not limited thereto.

The culture of the recombinant yeast can be performed using a normal method of culturing yeast, and the culture conditions can be appropriately set under conditions suitable for the growth of the yeast.

The culture medium used for the culture may be any of a natural medium or a synthetic medium as long as it contains a carbon source including sucrose, a nitrogen source, an inorganic substance, and a trace nutrient required by the used strain as necessary.

The carbon source of the culture medium may include sucrose and may include a carbon source other than sucrose. As the carbon source other than sucrose, for example, a sugar such as glucose, maltose, fructose, mannose, trehalose, mannitol, sorbitol, starch, dextrin, or molasses, an organic acid such as citric acid or succinic acid, glycerol, or the like can be used.

As the nitrogen source of the culture medium, for example, an organic nitrogen source such as corn steep liquor, soybean meal, or peptone, or an inorganic nitrogen source such as ammonium chloride, ammonium sulfate, urea, ammonium nitrate, sodium nitrate, ammonium phosphate, or ammonia can be used. Furthermore, a nitrogen-containing natural substance such as peptone, polypeptone, bacto peptone, meat extract, fish extract, yeast extract, corn steep liquor, soybean flour, soybean meal, dried yeast, casamino acids, or soluble vegetable protein can also be used as the nitrogen source.

As the inorganic substance of the culture medium, for example, a calcium salt, a magnesium salt, a potassium salt, a sodium salt, a phosphate salt, a manganese salt, a zinc salt, an iron salt, a copper salt, a molybdenum salt, a cobalt salt, or the like is appropriately used. Specifically, potassium dihydrogen phosphate, dipotassium hydrogen phosphate, magnesium sulfate, ferrous sulfate, manganese sulfate, zinc sulfate, sodium chloride, potassium chloride, calcium chloride, or the like is used. Furthermore, phosphoric acid, sulfuric acid, potassium hydroxide, Trace Element Solution (TES), or the like is appropriately used.

As the culturing method, a liquid culturing method is preferable, and any of batch culture, fed-batch culture, continuous culture, or perfusion culture may be used.

The culture temperature and pH may be conditions suitable for the proliferation of the recombinant yeast. The temperature is preferably 20°C to 40°C and more preferably 25°C to 35°C. The pH is preferably 2 to 9 and more preferably 5 to 8.

The culture time is preferably 1 hour to 14 days, more preferably 6 hours to 10 days, and still more preferably 12 hours to 7 days.

The culture can be preferably performed by shaking or aeration stirring.

In a case of secreting a non-secretory target protein extracellularly from yeast, a nucleotide sequence encoding a signal sequence can be introduced to the 5' end of the target protein gene. The nucleotide sequence encoding the signal sequence is not particularly limited as long as it is a nucleotide sequence encoding a signal sequence that can be secreted and expressed by the yeast. Specific examples of the nucleotide sequence encoding the signal sequence include a nucleotide sequence encoding a signal sequence of mating factor $\alpha$ (MF$\alpha$) of Saccharomyces cerevisiae, acid phosphatase (PHO1) of Ogataea angusta, acid phosphatase (PHO1) of Komagataella pastoris, invertase (SUC2) of Saccharomyces cerevisiae, PLB1 of Saccharomyces cerevisiae, bovine serum albumin (BSA), human serum albumin (HSA), and an immunoglobulin.

By culturing the recombinant yeast according to the embodiment of the present invention, the target protein can be accumulated in the host or the culture solution and recovered. As a method of recovering the target protein, known purification methods can be appropriately combined and used. For example, the recombinant yeast is cultured in a culture medium, and the cells are removed from the culture supernatant by centrifugation or filtration of the culture solution. The target protein can be recovered from the obtained culture supernatant by using a method such salting out (ammonium sulfate precipitation, sodium phosphate precipitation, or the like), solvent precipitation (protein fractionation precipitation method using acetone or ethanol or the like), dialysis, gel filtration chromatography, ion exchange chromatography, hydrophobic chromatography, affinity chromatography, reversed-phase chromatography, or ultrafiltration alone or in combination.

The present invention will be more specifically described using the following examples; however, it is not limited by the examples.

Examples

<Example 1> Cloning of MAL cluster

The genome of Ogataea Parapolymorpha containing the MAL cluster having SEQ ID NO: 1 and the genome of Pichia pastoris CBS7435 having AOX2 were used as a template.

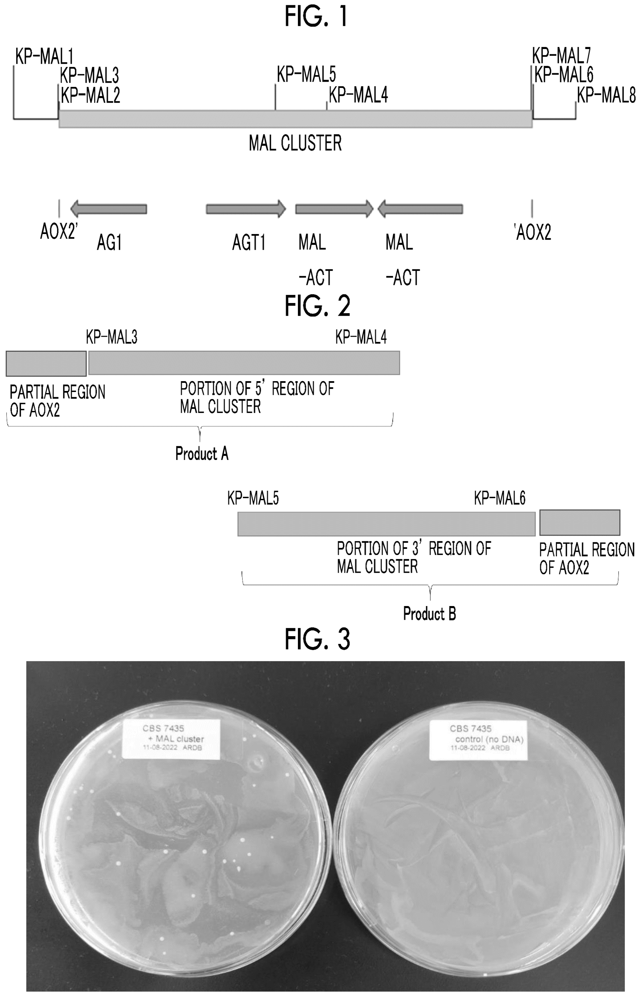

As shown in FIG. 1, using KP-MAL3 and KP-MAL4, the 5' region of the MAL cluster was amplified using the genome of Ogataea Parapolymorpha as a template. The nucleotide sequence of the amplified fragment is set forth in SEQ ID NO: 2. Using KP-MAL1 and KP-MAL2, the partial region of AOX2 was amplified using the genome of Pichia pastoris CBS7435 having AOX2 as a template. The nucleotide sequence of the amplified fragment is set forth in SEQ ID NO: 3. In addition, using the two amplification products obtained above, a concatenated amplification product was obtained by a PCR reaction (product A).

Similarly, using KP-MAL5 and KP-MAL6, the 3' region of the MAL cluster was amplified using the genome of Ogataea Parapolymorpha as a template. The nucleotide sequence of the amplified fragment is set forth in SEQ ID NO: 4. Using KP-MAL7 and KP-MAL8, the partial region of AOX2 was amplified using the genome of Pichia pastoris CBS7435 having AOX2 as a template. The nucleotide sequence of the amplified fragment is set forth in SEQ ID NO: 5. Using the two amplification products obtained above, a concatenated amplification product was obtained by a PCR reaction (product B).

The relationship between product A and product B is shown in FIG. 2.

Primers for MAL cluster amplification

KP-MAL1 (Forward):
5'-TTGTCAGCTTAAAGGACTCC-3' (SEQ ID NO: 6)
KP-MAL2 (Reverse):
5'-TTCCGGGAATACCATTTTTCTCAGTTGATTTGTTTGTGGG-3' (SEQ ID NO: 7)
KP-MAL3 (Forward):
5'-CAAATCAACTGAGAAAAATGGTATTCCCGGAAAGAACTCG-3' (SEQ ID NO: 8)
KP-MAL4 (Reverse):
5'-CCTGGTCTTCTGTGAGTATC-3' (SEQ ID NO: 9)

KP-MAL5 (Forward):
5'-TGGGCAGGCTTGTCTGTTTG-3' (SEQ ID NO: 10)
KP-MAL6 (Reverse):
5'-CATAGATACAACATAAACTAGGAATGTTTCCAGCGGTATG-3' (SEQ ID NO: 11)
KP-MAL7 (Forward):
5'-CATACCGCTGGAAACATTCCTAGTTTATGTTGTATCTATG-3' (SEQ ID NO: 12)
KP-MAL8 (Reverse):
5'-CTGACTGGACTTAGCGAAG-3' (SEQ ID NO: 13)

The product A and the product B (each sample amount of 2 to 10 μg) obtained above and Pichia pastoris CBS7435 (OD600: 200 to 300, 60 μL) were simultaneously introduced into Pichia pastoris CBS7435 by electroporation (MicroPulser electroporator (Bio-Rad)) to perform the transformation of Pichia pastoris CBS7435. As a result, the product A and the product B were inserted into the AOX2 site of the genome of Pichia pastoris by homologous recombination. To confirm that the MAL cluster composed of the product A and the product B was introduced into the CDS of the AOX2 site by homologous recombination, the screening was performed using a YNB-sucrose-supplemented medium, and a colony in which the MAL cluster was incorporated was acquired (FIG. 3).

<Example 2> Production of plasmid vector in which gene of target protein is linked downstream of sucrose-inducible MAL promoter

As a model protein, recombinant gelatin CBE3 described below was used. (Described in WO2008/103041A).

Molecular weight: 51.6 kD
Structure: $GAP[(GXY)_{63}]_3G$
Number of amino acids: 571 amino acids
RGD sequence: 12 sequences
Imino acid content: 33%
Almost 100% of amino acids have a repeating structure of GXY.
CBE3 has an ERGD sequence.

The amino acid sequence of the CBE3 does not include a serine residue, a threonine residue, an asparagine residue, a tyrosine residue, and a cysteine residue.

Isoelectric point: 9.34
Hydrophilic repeating unit ratio in polymer: 26.1%

The amino acid sequence is set forth in SEQ ID NO: 14.

(SEQ ID NO: 14)

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)3G

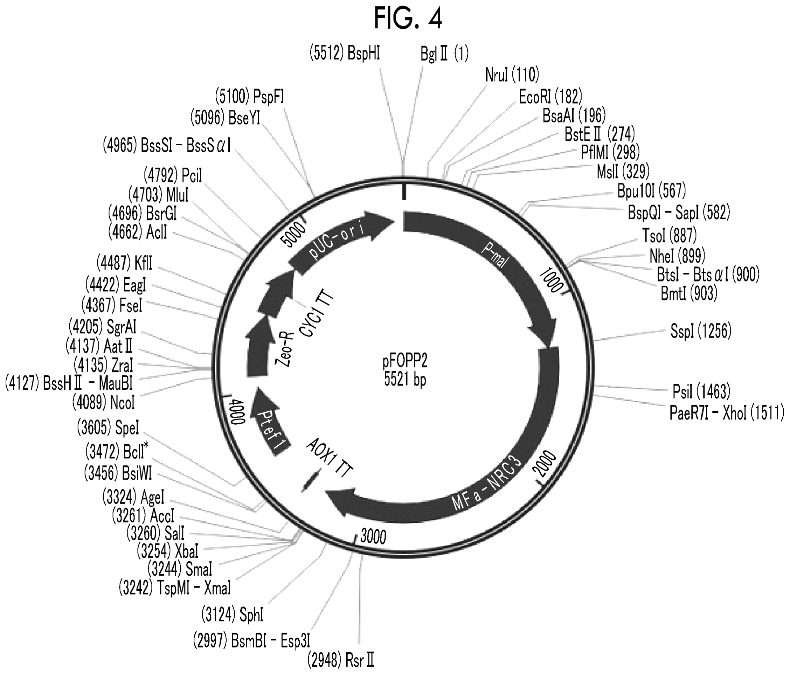

A plasmid vector in which a gene encoding a target protein and a zeocin resistance gene were linked to the downstream of the sucrose-inducible MAL promoter was produced.
The nucleotide sequence of the produced plasmid vector is set forth in SEQ ID NO: 15.
The configuration of the produced plasmid vector is shown in FIG. 4.
The nucleotide sequence of P-mal is set forth in SEQ ID NO: 16.

MF-a: signal sequence of target protein is shown in SEQ ID NO: 17.
NRC3: nucleotide sequence encoding target protein (CBE3) is set forth in SEQ ID NO: 18.

<Example 3> Transformation of Pichia pastoris

The plasmid vector (plasmid vector having a sucrose-inducible promoter) produced in Example 2 was introduced into the Pichia pastoris CBS7435 strain produced in Example 1, by homologous recombination. Specifically, the plasmid vector (produced in Example 2) of SEQ ID NO: 15 in which a gene of a target protein was linked to the downstream of the sucrose-inducible MAL promoter was linearized with a restriction enzyme EcoRI. The linearized plasmid (amount of linearized plasmid of 2 to 10 μg) was introduced into the Pichia pastoris CBS7435 strain (OD600: 200 to 300, 60 μL) produced in Example 1 by electroporation. The electroporation was performed using an electroporation device (Micro-Pulser electroporator (Bio-Rad)).

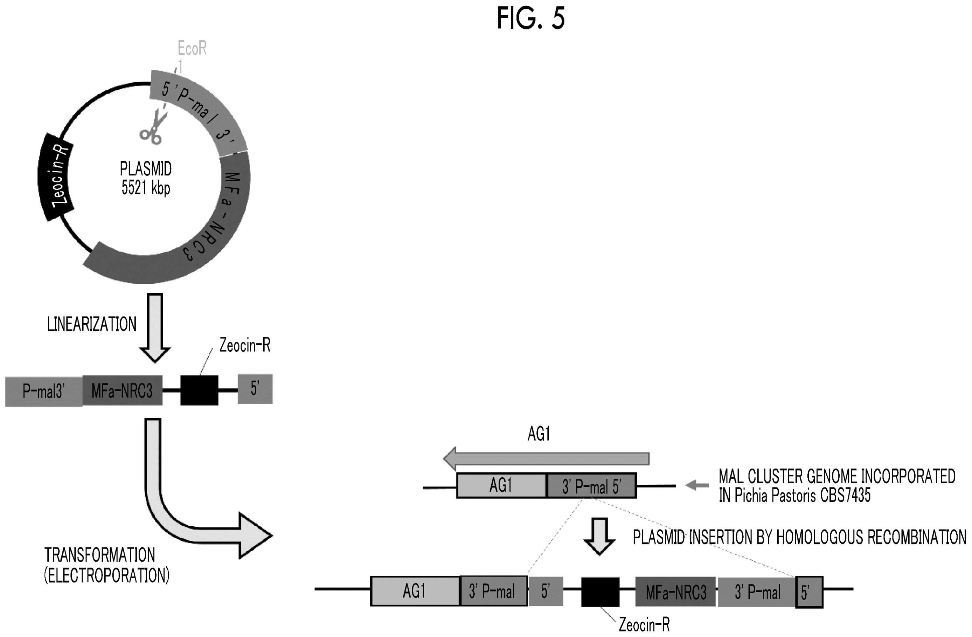

The Pichia pastoris CBS7435 strain transformed by electroporation was seeded on a YPD agar plate containing an antibiotic, zeocin (500 μg/mL) as a selection marker, and screening was performed to acquire a proliferated colony. As a result, as shown in FIG. 5, a transformed yeast in which an expression unit containing a MAL promoter and an NRC3 (CBE3) gene was incorporated into a sequence of the MAL promoter in the expression unit containing the MAL promoter (also referred to as P-mal) and an AG1 gene was acquired.

FIG. 5 shows a schematic diagram illustrating that the linearized plasmid is inserted into the AG1 site on the MAL cluster by homologous recombination.

<Example 4> Culture and protein expression

To examine protein expression, the transformed yeast produced in Example 3 was cultured using a 3 L fermenter. 9 mL of the yeast culture solution obtained by the 24-hour expansion culture was collected, and 23 mL of Biotin and 5.1 mL of an antifoaming agent (struktol J673, Schill and Seilacher) were added to the batch medium 900 mL (Batch Medium 225 mL + water 675 mL) in the jar at the same time to start the fermentation. 24 hours after the start of the fermentation, 5.1 mL of an antifoaming agent (struktol J673, Schill and Seilacher) and a sucrose aqueous solution were added. 3.6 mL of TES was added at each of 24, 48, and 72 hours after the start of the fermentation. Ammonia was automatically added (cumulative amount of about 130 mL) by receiving feedback from a pH sensor, as a nitrogen source and for pH adjustment. A total of 970 mL of sucrose was added from 24 hours after the start of the fermentation to the end of the culture.

$$\text{Sucrose addition rate: L (mL/min)} = 0.262 \times e^{0.01t} \text{ (e is natural logarithm: 2.718)}$$

t is time (hr) from the start of the fermentation.

The pH was maintained at 5.5, and the pO2 was maintained at 25% from 2 hours after the start. The fermentation was stopped 100 hours after the start of the culture, and the culture solution containing the produced protein was recovered.

[Table 1]

| Solution composition | |
|---|---|
| Name | Composition |
| Batch Medium | phosphoric acid 163.4 g/L<br>sulfuric acid 2.5 mL/L<br>potassium hydroxide 36.0 g/L<br>TES 17.4 mL/L<br>calcium chloride dihydrate 2.0 g/L<br>magnesium sulfate heptahydrate 37.4 g/L<br>glycerol 240.0 g/L |
| Aqueous ammonia | 25% to 28% (w/w) aqueous solution |
| Antifoaming agent | 10% Struktol J673 |
| Sucrose aqueous solution | 50% (w/w) aqueous solution |

The expression level of the expressed protein was evaluated from GPC analysis (equipment used: Waters e2695 HPLC alliance). The results are shown in the following table (column of sucrose-inducible promoter-introduced yeast in the following table).

As a comparative example, the measurement result of the expression level of expressed protein in a case of using the methanol-inducible promoter-introduced yeast is also shown in the following table (column of methanol-inducible promoter-introduced yeast in the following table).

<Comparative Example 1> Production of methanol-inducible promoter-introduced yeast

A plasmid vector in which a gene of a target protein (CBE3) and a zeocin resistance gene were linked downstream of a methanol-inducible AOX1 promoter was produced using a pPICZα plasmid vector (Invitrogen).
The nucleotide sequence of the produced plasmid vector is set forth in SEQ ID NO: 19.

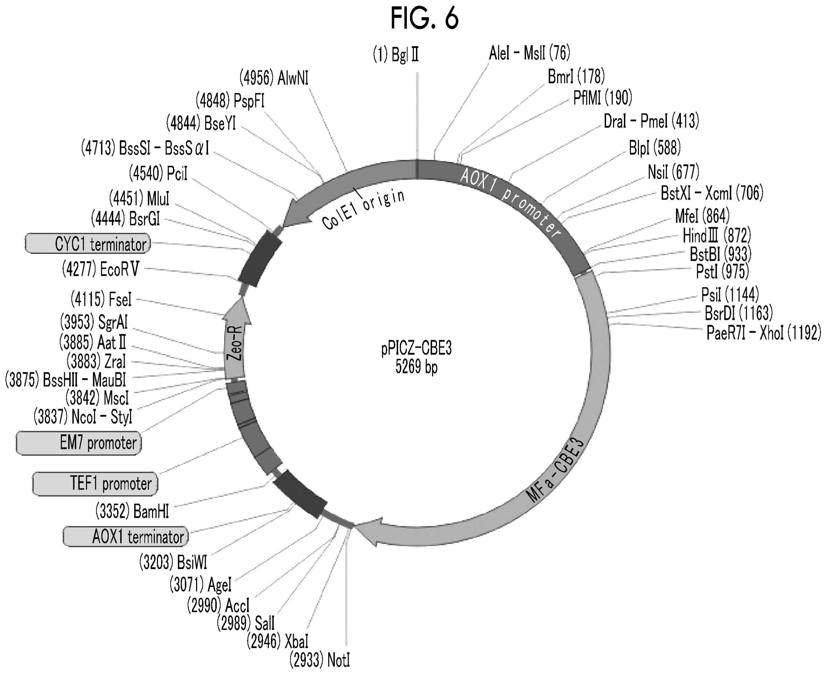

FIG. 6 shows a configuration of the produced plasmid vector.
The Pichia pastoris strain (X-33) transformed by electroporation was seeded on a YPD agar plate containing an antibiotic, zeocin (500 μg/mL) as a selection marker, and screening was performed to acquire a transformed yeast strain from a proliferated colony.

<Comparative Example 2> Culture and protein expression

To examine protein expression of the methanol-inducible promoter-introduced yeast, the transformed yeast produced in Example 0090 was cultured using a 3 L fermenter in the same manner as in the sucrose-inducible promoter-introduced yeast. 8 mL of the yeast culture solution obtained by the 24-hour expansion culture was collected, and 23 mL of Biotin and 5.1 mL of an antifoaming agent (struktol J673, Schill and Seilacher) were added to the batch medium 900 mL (Batch Medium 225 mL + water 675 mL) in the jar at the same time to start the fermentation. 24 hours after the start of the fermentation, 5.1 mL of an antifoaming agent (struktol J673, Schill and Seilacher) and methanol were added. 3.6 mL of TES was added at each of 24, 48, and 72 hours after the start of the fermentation. Ammonia was automatically added (cumulative amount of about 100 mL) by receiving feedback from a pH sensor, as a nitrogen source and for pH adjustment. A total of 850 mL of methanol was added from 24 hours after the start of the fermentation to the end of the culture.

$$\text{Methanol addition rate: L (mL/min)} = 0.229 \times e^{0.01t}$$

(e is natural logarithm: 2.718)
t is time (hr) from the start of the fermentation.
It is noted that the methanol addition rate was set to be the same as the carbon equivalent per unit time of the sucrose addition rate.

[Table 2]

| Yeast | Yeast wet weight (g/L) | Survival rate (%) | Expression level of protein (g/L) |
|---|---|---|---|
| Sucrose-inducible promoter-introduced yeast | 640 | 100 | 6.8 |
| Methanol-inducible promoter-introduced yeast | 117 | 8.8 | 0.05 |

In the sucrose-inducible promoter-introduced yeast according to the embodiment of the present invention, a protein expression level higher than that of the conventional methanol-inducible promoter was obtained. In addition, in the methanol-inducible promoter-introduced yeast, the survival rate was decreased by adding methanol, but in the sucrose-inducible promoter-introduced yeast, there was no effect on the survival rate by adding sucrose, and continuous protein expression was suggested.

(SEQ ID NO: 1): Sequence of MAL cluster

GTATTCCCGGAAAGAACTCGGGATACGGCCGGATTATTAGCTAAACGAGC
CATGTTAGCAAAACGGTTGGCGAGGTTTATTTTTTTATTTACATTTCACTGCAAGT
CGATCGACCGTGTCGCGACTAATGAATAATGAATATATTATGAAATGTCAAGATT
CAACTAGTCTATCCCAATCTGGCTCCGCAAACGCTTCGACTGTCTCATAAACAAA
GAAAATATTCAACTATATACCTTTATTAGGCTGTAAAATCTAATTGACCTCGATCA
GTCTACCCTCATATGGTTGCAGCTTGCCTGTGATGTTGTTTGCAATATTAGACAGG
AGCAGCTTTCCTTGAGGGATTGGGTACTCGACTTCTCTATCAGTAAAGTTCAAAAC
AACAGCCATCTTCTTTTGGCCTTTGTTGCTGGTCTTGGTGTAGTAGAAAACCTCCT
GGTTATCGTAGTCTTGCACCTCGAAACGGCCAAAAGTCAACGTCTCAGGATAAAG
CTTTCTGGTCTTGATGGCATTTCTGTAAAGTTGAGCACAGAGTTAGGGTCATCTT

TCTGCGAAGCAACGTTGATGTCTTTGTAGTTGTCGTTGATTCTCATCCAAGGCTTA

CCACCGAAACCACCGTTTTCAGAAGAGTCCCACTGAACTGGAGTCCTTGCATTGTC

TCTTGCCAGCAAATTGATGATCTTCAGCAGCTCGGCCTTCTCCTCATCAGAGTGCT

CGGTCTCGTTGAACGCGTTCCAGTAGTTAATGGTGTTGATATCCAAATACTCTGAA

ATATCCCATTTCGGTGAAACATTGTCATACCAATCTCTTGACCTTGGTACACAAA

CAGAGTGCCGGTCAAGGTTGTCTGCAACAGTGCCAGCATCTTTGCAGATCTGCTCC

AAAACAGTTTGTTGGAGGTGTTTCCGAACCTTGTGACACAGCGTGGTTGGTCGTG

GTTCTCGATAAAAACGGTGGACCATGCGTCATTCAGCTCACCGGTCTCGTCATCAA

AGATGAATTCTGACTGGTTGATGATGGCATCCTTGAAGTCGGTCAAGTTGAAGCC

TTTGTATCTAAAACGGTCGCTCTTGTCGGATCCAACGTCAACAGTGTCGAACAGG

AACATCATGTTCATCTCCTTTTCTTTTGCAGAGACGTACTTGAGAGCATCTGCCTT

GGAGCAGTGACCAACTTCTCCGACAGTCATGGCATCATACCTGGAAGTCACTTTCT

CGTACATCTCTTTGTGGAACTCGTGAATACGTGGACCGGAGTTGATGAGTGGACC

AGCCGGCTGGTATTTCTCACCTGGGAAGGTGATTGGCGCATCCTCAAAAGTCTGG

ACTTTTGAGTAGAGGCCAGCAGTGTCGATTCTGAAACCAGAAACGCCCTTCTCGT

ACCAAAACTCAAGAGCAGATTTGTAAATGGCTTCACGGGTCTCTGGGTTCTCCCA

GTTCAAATCAGGCTGAGTCTCAGCAAACAGGCGAAGGTAGTATTGCTTAGTCTTTT

CGTCATAAGCCCATGCAGAACCAGAGAAAAAGGAAAGCCAGTTGTTGGCATTGTC

CTTCCAAATGTACCAATCTCTCTTTGGATTGTCTCTGCTAGATCTAGACTCCTTGAA

CCAAGCGTGCTCAGACGAGGTGTGGTTAATAACGAGGTCCAGAATCAATCTCATG

TCTCTCTTCTTGATCTCGGCAAGCAGTCTGTCCATGTCCTCCATAGTGCCAAATTG

AGGGTTGATGGCATTGTAGTCAGAAATATCGTATCCCATATCGTCCAATGGCGAA

GCATAGTGCGGAGAAAGCCAGATCACATCAGTACCAAGGGATTTGATATGGTCGA

GCTCGGAAGTGATTCCGTTTAAGTCTCCAATACCGTCTCCGTTGGAGTCTTTGAAG

GAAGCAGGCCAAACTTGGTAAACAACGGCGGATTTCCACCAAGGTTCTTGAGACT

CGATAGTCATAAGGAATTAATATTGTCAAGAGGGATAAAAAAATTGTGGAGGCG

GAGGGTCTTTATATGGCTTGAGCTTACTAAAGACATTCTCATCACTTTAGCGAGCG

CGACGCTTTGGTATCCTCCTAACTTCCCGAAAATAAAAGAGGATTTCCGTTTGCG

TCATGGGCTCCACCTCCTATGCACGTCCATAATAGTTATTTGATTGTTATCTGTACC

CCAGACAAGATCCTGTTTCAGCGAGTATTAAATCACGGCCTAATTTATTTGAGCGA

GAATTAATTTGCACATTTAATTCTCGCTGAAATAACTGCCAACAATTGAGTCTGGG

GTACATGAATAGAAGTGACGTAATTGACCCCCACGCTTGCTAGCCACTGCGGAGT

CACTTGAACCACATGAGAGCCCGTAGGGAAGCCAGAGCAATCAACGTCAAAGGG

CTCTTAGGAAACTTGTATAACCCAAGTGTTCATTACGACGCTGCAGAGCTCTCACA

TTATATGTAGAGTGGTACGGCAGAGTCTGTAGAACATACAAAGCTATCATGTCGC

GTAATCTGCACAGTGCCTGCTTTCTATTGCGATACTCCCGAAGATATCCCTATCCG

CATACACGGAACTGTAGTTAATTTGCCGTGGAGTATCAATGTTTGTCCAACGCGCC

GAAACTCTTTTTGCGGGGCTCTTCTTCATCTAACTCGTCCTAAGCTGGCTCGGGGT

ATCGCATAAACGTCTGCGTGCATTGCCAAGTTGTTTTGGTCTGTGTTGAACCTTCT

TTCCAAGCCCCATCCCGAACCCCGGAAAATTCCCCGATTATTATTTCCCCGCAAAT

GCGGCGTGCCAGAATCTCTCAGCCTTGCGTCATAATTTAGATTTCGCAATTATTTC

GTGGAGCAATAGGCCGCGAGCGTCCTATTGTGCTGTTGGCTCGAATCTCATGCAC

AAAAATGAGGATTTGCCAGAGTGTGTTTTCCAAGGCGTGGCCAAAACAGAGGGTC

ACCAGACAACAGAGCTCACCATAATACACAGCTTTATGGTCTTACACTAAGTCCT

CCTCCTCAACAACGCGGGGCTATACGTGTTTGCAGAATTCTTCCATGCGTGATTTG

CAATGGTGCTTGCATACTGCAACAATGCGTATGGGGTAATTAAAACGAGCACTCG

CGAGCATTCATCAAAGGTAAAAAGCATTATTCTAAGCTCTTTCTGGAAGCAAACT

TGGGCCGCGATCACGCACCAAAATTCAGCCGGATTAAGAATGGGCAAGCATCTCC

CCACGTCATATTTGTGTGGCATTAGGACTATATATAGTTGATATATTCTCGACACA

CTAGTATCCATCAGCATGCCTGAGTTCGTCGAAAACATCGAGAAGCCTGAAGAAG

TGGAAGTCATTCCAGACATCACAAAGAAAATTAACACTCTTTCTGACAGTGATGA

TGGAAGTGGTGCTTTCAACGACTACATTGCCAGATTCGTGGAGATTTCGACGAAT

GCCCAGAATAATGAGCATCAAGAAAAACACATGTCGCTGAAGGAAGGTCTGAAA

ACTTTCCCGAAAGCTGCCTGCTGGTCGATTGTGCTTTCCACAGCCATCATCATGGA

AGGATACGATACCATGCTCTTGAATAGTTTGTACTCGATGCAATCTTTCGCCAAAA

AATACGGCCAGTACTACCCGGAAATTGACGAGTACCAGGTCCCTGCCAAGTGGCA

GACGTCGCTTTCGATGTCGACTTATGTCGGTGAAATTGTCGGACTGTACATTGCAG

GTCTTGTTGCCGAGAAATGGGGATACAGACGTACGTTGATATGCTTCATGGCAGC

CGTTGTGGGTTTAATCTTCATTCTTTTCTTTGCGGTCGACGTGCAGATGCTGCTTGC

CGGCGAGCTGCTCTGTGGTATTGTCTGGGGTGCATTCCAGACTCTTACAGTGTCAT

ACGCCTCTGAAGTCTGTCCCGTTGTCCTGAGAATCTACCTTACCACTTATGTCAAT

GCGTGCTGGGTTATCGGACAGTTAATTGCTGCTTGTTTGCTGAGAGGCACTATGAC

CCTGACCAACGAGTGGTCATACAAAATTCCTTTCGCCGTTCAGTGGATCTGGCCTG

TGCCAATCATGATTGGAATCTACCTGGCCCCGGAGTCGCCCTGGTGGCTGGTTAA

AAAGAACCGGGACGCAGAAGCCAAGAGAAGTATCATGAGACTTTTGAGTCCAAA

CACCGAGGTGCCAGACGTTGCTCCGCTAGCCGAGGCAATGCTGAACAAAATGCAA

CTAACCATCAAGGAAGAGTCTGCGCGCACATCCAATGTCTCGTACCTTGACTGCTT
CAAACACGGCAACTTCAGAAGAACAAGGATTGCCTCCATGATCTGGCTCATCCAG
AATATCACTGGATCTGTCTTGATGGGCTATTCGACCTACTTTTACATCCAGGCAGG
ACTTGATAGCGGTATGTCTTTCACTTTCTCTATCATCCAATATGCACTGGGTCTTCT
TGGAACAATTGCCTCGTGGCTGCTGTCGCAAAAAATGGGCCGTTTCGACATCTATT
TCTTGGGTCTTAGCATAAACACATGCATCCTGATAATTGTCGGAGGTTTGGGCTTC
TCTTCATCGAGCAGTGCGTCTTGGGCAATTGGTTCGTTGCTCCTTGTCTTCACTTTT
GTCTACGACTCATCCATCGGTCCTATCACCTACTGTACGGTTGCAGAAATCCCTTC
GTCAACGGTGCGTGCCAAGACAGTTGCCCTTGCAAGAAACTGGTACAATCTGTCT
CAAATCCCGCTCTCCATTGTCACTCCATACATGCTGAATCCGACTGCTTGGAACTG
GAAAGCAAAAGCAGCCCTTTTATGGGCAGGCTTGTCTGTTTGCTCACTGATCTACA
TCTGGTTCGAGTTCCCAGAAACAAAAGGAAGAACTTATGCTGAACTGGACATCCT
ATTCAAAAACGGTACCAGTGCCCGTAAGTTTAAGTCCACTCAGGTGGAAACATTC
AATCCTGAGGAAATGTTAAAAAAAATGAATAATGAGGATATTATACAGGTTGTTG
ATGGCGACCTGGATGCCGGTGCGGCCACTGCTAAAGTTTAAAAGTTGAAATGTAT
AGTTTCAGTTCTGCGATGTTATTATTTTACAATTGAATATGCTATCTCGAATGATTT
TTTGTAATAGCCGTAGATGTCTTTATTATTGCATTTAAGGGTTCAAAATTATTCAT
GTGAGCGTTTTATTTTACGGGACAATAAGATTTTGGGCGAGACTTCCCCACAAATT
GAATAATGTGCAATATTTTTAAGAACTAAATTTACTGGAGACAATTTCTATGCCTC
TCAGTACACGTGCAAAACCACAAACGCCTGGCCAAAAGTCCCATTCTAAAAGGCC
GTATATCAGGCCTTGTGACGCGTGCGCGTTCAAAAGAGTACGTTGCGATGCTGAG
TTCAAGTTGGATCGCAGATGCACCAACTGTTTAGTTCATGGAATTGAGTGTACGA
ATAACAGAGTCAAGCAGAGATCTGGACCCAGAAAAATACATAAAAAAACTGAAG
AGGCTATTAAAAGCCTGGTAGAAGGAAGTGGCGCGTTCAGCAATCTTCGATTAGC
CACTCCGGCCCACAACATATGTGGTATTATCAATCCTGCCACCTGCGAGAAGAGC
TCAATATCGCTTAGTGAGATTCGACCGTACCTCAGAATTTATAAATCCCGATACTA
TGCACTCTGGCCAGTCCTGTCTGTTGATGACTGTCTGAATATTCTTAATGAAAGCT
CAGTTCACATAAACGATAGCGCATTTTTCATGCTCAATGCCAACAATGCGTCGTTG
TACGCTCTTTCATGCGCCTTGTGCGCTGTAATTGCAAGACACCACCTTCTGGTC
CCTGGAAGAATTTGATACAATGGCTCATTTAGGACTCAAAAGCTGCCCTCCGCCA
GAAACTTATGTAAGGGAGGCAGAGCGAGCTATCTACATGTTTGACCTTGACGGGA
TACTCACAGAAGACCAGGTTTTAACTAACTTCTTTCTGCATATATATTTTTCATCCC
TTGACGAGGACAACCTGCAGGAATTGGTCTATCTCAGACAGGCAATCAGCTGCGC

ACAAATGCTTCACCTTCGAGATAACGAAGTTTTGGGAGAATCACCAAAAGATACA

TCACACAGATTTAAAAAAATTTACTACTTACTTCTCATCACAGAGAGGTATGTCTC

GTTCAAAAAACAACTACCAGTGATATTAGAGCCAACAATGCCACTTCCAAGTTTG

GAAGATGATGAAGCCCCGGATCTGCTACCTGGATTTATCGAACTTGCGCAAGTAT

TCAGTGTTCCTGATTGCGCTTTCTTTAACAAGTACTCGCTCCGTGGCACGACAGCT

GCACTTGATCCTTTCAGCGAGAGCTTTGACCTAATGTTCAAAAACGAATGGATCC

AAGACGTGCAAACCAAGCTCTCTAGGTCGGTCGTTCAGGTGCGCAACCAGTCTCA

AAAGGTCAATATATTAATTTCTCGAACATGGTTGCAATCAATTGCTTGGTTAATGG

CCAAAGATCGACTTCTTCTATCGCCTACGAGTGATCAAGCCAATTTCTTTGATCCA

AGGTATCCTATTCATATCGCGAAAGACTTTCTGATACAGACTAAAGACATGCCGT

ACGAGGCATTTGAAACAAACGGTAGTGGTGTCATCGTGAAACTAAGCGAAGTCGC

CAACGCCTTGCAAACCTTCATCCGATTGGCTCCAGGATCGCCCGAAACAGCTTTA

GCATTAGACGAGCTTGCATATATCCACGGTATTATCATGCGGCAGAACTCCAACA

GCTCGCTCGGGCCTCTTATACACCTATCACTGAAACTTTGGAATCTCGCAGGTTC

GGATTTTATCGAAATGGCTGCGACATTTCTTATCCAAAAATAGAGTCTGCAGATTA

TGAAGAGAACGAAAATGACCATACACCCGAAATCATTGCCTCCATGCCGGAGAAC

TTGAATTTGACACCTCTTTTGAGCGAGTTCGAATTTCCGAGGTGAACAAGTATTAT

CTGCTCACGTGTTTGGTGCATTTTGCAACTAGCGACAAATTTCACTGGCATATTAG

TATATCATCAAATCATTAGGATTGCTATTTTGTGTCCACTAAGGCGCTCCAGGTGC

GTGGAATGGTGCAGTATTAGGCCCTAGCTGTCCGAGAAAAAAGGAAGCTACACCC

TTTGATGTAGTGGGCTCTTTGTGTGTTGTGAGAGGATTAAATTGGCCAGATGACAC

TCTTTCCAAAGATGCAAATAACGAGGAAGAAACATCCAGCCGGATGCCAGATGTC

TGTCTGTGTTGCAAATTCGCTCTTGATCCGGAAAAAGAGTTATTGAAAAGGGCAC

TGTCCTGTCTCGTATTGCAGCGTACGAGTGAACCTGAGCGTGTCAATGCAGGTTCA

TAAGAAGAAAATTCAGAAGACAGATTGTGACATATTTCTCGCAAGCCATTCACTT

TCCAAGATTCTAGGAGCTTAGAGATCTTGGTGAACAGGGCTCGGGGCAAGTCCAT

TCCAGCTTTGTACTTCAACACCATGTCGAATACGGCACCCATGCGTTCGTAGGCTA

GCTCGGTGGTTGCTGGGTCAACATAGCTTTTGATCAGAATGTAAAGAGAGTCTGC

TATCTCCAGTAATTTCATTGATATGTCGGGTCCGTTGCATCTAAAGGCATGATCAG

GGAGGTTTTGTAGACTGCTGAGAAGCTCATTTGCAACTTTTAGAGGAAAGTTAAT

CTTGAAACATTCGTGTTCGACCGCAATGCTCTCATCAGAACTAATTAGAAAATTCG

CAGACGCAATTAACCAGCCCAAAGACTGGAGCCAGGCTTTTGAGACAATAACATT

CACTTTTTGAGCACTTGTTGCAATTTTATCCACATCAATTCTAATTTGGAGCTTCTG

TTGGAGCTCAAGGAGCCACAGCCTCTTCAACTCTGTGGATCCGTTCAAGGTAAAA

TCCTGAAACAAGTTGGTCTCTGGAATCTGGAACATGTTGTGAGTGCGCCCTGTCTC

GAGCACTTTAGTATGAAGCTCAACATAGAATTGCTTATCCGTTGCACTGAAAATTG

CACACAGAGTGGTGAACCCGTAAAGTGAATTTGAGGATTTTTCGAAATCTGCTTC

AGGGAGTGGAATTGAAGGTTCAAGAAGAACGGGCAAATTAGTTTTAAAGCTGAC

AAACCTTTCGGTTACCACGAGCAAATAGTAGATTTTTTTCTGCCTATGAGCCTCAA

CGGGTGACAAATGAAGCAGTGACTGTGGTTCGTGCATTTTAAGATATCTGCAAG

AGAAACAGCTTCACGGAGGTAAATAAGACCTTTGTGCTGTTGGCCAGGCGCAATA

GCATAGTATTTATGTAGGAAGAAGGATGTCAGAAGGAGATCAATATTTGGTGTGC

TTTTCAGTCTATACTCATAAATTATCCTCCTTGCCTCTTGAGCATATAAGCCAGCAT

TTTTGAGATCGCTCCTGGCTGGAGTGTATGTGACGAAACTGTGATATTGTGCGATC

GTAGCGCACAATGCGCAACAAAGAGCATATTCTTGGGCATTGTCCTCGGCGATGG

TGATACGAAAATCATCTAAAACATTCGCTAGTTGAAATACCTGCTCCACATTCTTT

AAGGGGCTTGAACGTGTCAAAATTGAAACTGTACTTTGAATTGATACTACCGGCC

AGATATCGTAAAAATTCGAATGATATCTCAAGGTAAGGGATAATTTGTTTCAA

AGAAAATGCGCACACATGAGCAGGTAGTGTAGACTGAGCATAGATTTCGTCGTTG

AGATCCTCTGTGTTTTGACTGTTCTAGTACTTTTCTTTGGACCGGATTTATGCCTC

ACGCGGAGGTTGGTACATGGAACTTTATGAGTGAGACAGTTCGAGCAAATACCAC

CGGTGCATGAATCTATATCGCACTTCACACGGCGGATGGCGCATGCGTCACAGGG

TCTCGGCCGTTGTCGTTTTTGGTGGACGGCTGCGACATCTCGGTATTTAGATAAG

CCAGCTTGTACTTGTATCCAAAATGTGGGGCTAAAGGGGAGTGTATGATTTTTAAT

GATATCGGTGAATGTCCAGAAATTTGGTAAATCGCACACCAAACTTCAACGATGG

ACTAGCGTGCTAAACTGATATTCTACCAAACAACCGTTTGAGTAATTCTGACCATG

ACAGAGGTCTTGTGTATATATTTTAAGCCCCGTCGATCGACCGCATCGCGACTCCA

TAAAAAAATTCATTTCACGCGTTATTATTATCGTATGAATATGGATCCCCTAAACG

TGAAAGTGCAACAGAAACTTAAGGAGCTCGAAAGCCTTCAGCAGATAAGAGACT

TGACGAAACACCTGAATGTTTCACTGGAAGAATTTGCTGGCCAAATAGAACTTCT

GGGTGAGGAAGCAGGATGCATCGAAACCGTGACGCAAACTGGATGAGAATCAT

CAGAGCAGTGAGCCTAGCCTCTAACTCTTTGTCGAACTACAAGGAAGAGGACTAT

GAAACGGATAGACCAATGACGGAGCGCCTTGTGAGATGCAAGATAGACGAGAGT

CAAAAAATCATCACCAAAATTGACTTACTTCAATCTATCTAACCATTGTTCTAAA

TTGATCTTGTTGTTCAGTTCCGACAACAGATCGTTCTCCTTCAGCAAATCGTCACG

AAGGCCTTTATCAGCCTCGAGATAATTAATATCGTCCGGATCTAACATTAAGTCCT

GAACTTTAGAGGGAAGTTCTATTTTGAGCGTCGATAACCCGGTCATCATTTTGAAG
TATTCATTAGTATGCACGTCAGAACCATTCAAAAGCCCATCCCAATCAGTTGTCAA
AAACAGCCTTTTCCATATCAAGGTTTCCAAGATTTCGTGATAGTGCGAATAACCAC
TCAGGTTACTTATCACTCTTGACGGATTGGAGTGTTTGATGACAGTCAACAATTTG
AACAAGTTGGCGAAATTGTTGTGACTGAATTCATCAAACGTCACTAATGTCAGTA
GCTGGACCATCTCAAACAATCCCAGCTGTTGCTGCTTATCCATCTTTGCCACAATA
TCGCCGATCTGACTTTCAAAATGTGTAAACTTGTTATCAACCAAGTATCTTGAGTC
TTGTATGAATCCATCTTTAGTTTTTGAATCAATTAATCTCGCATAATTCAATTGAA
GCTCAATCATTTGCAAGTGTATTTCTATCTCATCCAACAGATTGTCGTGATCCTCA
TTCAAAGCTATCAGTGAAAGTTTGGCA

ATATTCAGTTCGAACTTTTTCTTTTCAATTTCCTCCGACTCTGCAGTGGAA
ACATATGCAATCAGCTTTTCTGCTGCCTTGAAATACTCATCGTTTTCCACGTCAAG
TATCCAGGCAAATTTATAGTTGTTGATGCTTGAATTCAAAAACATGCGCACGTAAT
CTGGATATTGAGGGATGTAATTGAGGATTAGCCGAATCTTTCTGGTTCTAGCATAG
AATCCAAAAGCACTTCTGCAAAACGGTATCCATACCGCTGGAAACATTCC

(SEQ ID NO: 2) (fragment amplified with KP-MAL3 and KP-MAL4)

CAAATCAACTGAGAAAAATGGTATTCCCGGAAAGAACTCGGGATACGGC
CGGATTATTAGCTAAACGAGCCATGTTAGCAAAACGGTTGGCGAGGTTTATTTTTT
TATTTACATTTCACTGCAAGTCGATCGACCGTGTCGCGACTAATGAATAATGAATA
TATTATGAAATGTCAAGATTCAACTAGTCTATCCCAATCTGGCTCCGCAAACGCTT
CGACTGTCTCATAAAACAAAGAAAATATTCAACTATATACCTTTATTAGGCTGTAA
AATCTAATTGACCTCGATCAGTCTACCCTCATATGGTTGCAGCTTGCCTGTGATGT
TGTTTGCAATATTAGACAGGAGCAGCTTTCCTTGAGGGATTGGGTACTCGACTTCT
CTATCAGTAAAGTTCAAAACAACAGCCATCTTCTTTTGGCCTTTGTTGCTGGTCTT
GGTGTAGTAGAAACCTCCTGGTTATCGTAGTCTTGCACCTCGAAACGGCCAAAA
GTCAACGTCTCAGGATAAAGCTTTCTGGTCTTGATGGCATTTCTGTAAAAGTTGAG
CACAGAGTTAGGGTCATCTTTCTGCGAAGCAACGTTGATGTCTTTGTAGTTGTCGT
TGATTCTCATCCAAGGCTTACCACCGAAACCACCGTTTTCAGAAGAGTCCCACTGA
ACTGGAGTCCTTGCATTGTCTCTTGCCAGCAAATTGATGATCTTCAGCAGCTCGGC
CTTCTCCTCATCAGAGTGCTCGGTCTCGTTGAACGCGTTCCAGTAGTTAATGGTGT
TGATATCCAAATACTCTGAAATATCCCATTTCGGTGAAACATTTGTCATACCAATC
TCTTGACCTTGGTACACAAACAGAGTGCCGGTCAAGGTTGTCTGCAACAGTGCCA
GCATCTTTGCAGATCTGCTCCAAAACAGTTTGTTGGAGGTGTTTCCGAACCTTGTG

ACACAGCGTGGTTGGTCGTGGTTCTCGATAAAAACGGTGGACCATGCGTCATTCA
GCTCACCGGTCTCGTCATCAAAGATGAATTCTGACTGGTTGATGATGGCATCCTTG
AAGTCGGTCAAGTTGAAGCCTTTGTATCTAAACGGTCGCTCTTGTCGGATCCAAC
GTCAACAGTGTCGAACAGGAACATCATGTTCATCTCCTTTTCTTTTGCAGAGACGT
ACTTGAGAGCATCTGCCTTGGAGCAGTGACCAACTTCTCCGACAGTCATGGCATC
ATACCTGGAAGTCACTTTCTCGTACATCTCTTTGTGGAACTCGTGAATACGTGGAC
CGGAGTTGATGAGTGGACCAGCCGGCTGGTATTTCTCACCTGGGAAGGTGATTGG
CGCATCCTCAAAAGTCTGGACTTTTGAGTAGAGGCCAGCAGTGTCGATTCTGAAA
CCAGAAACGCCCTTCTCGTACCAAAACTCAAGAGCAGATTTGTAAATGGCTTCAC
GGGTCTCTGGGTTCTCCCAGTTCAAATCAGGCTGAGTCTCAGCAAACAGGCGAAG
GTAGTATTGCTTAGTCTTTTCGTCATAAGCCCATGCAGAACCAGAGAAAAAGGAA
AGCCAGTTGTTGGCATTGTCCTTCCAAATGTACCAATCTCTCTTTGGATTGTCTCTG
CTAGATCTAGACTCCTTGAACCAAGCGTGCTCAGACGAGGTGTGGTTAATAACGA
GGTCCAGAATCAATCTCATGTCTCTCTTCTTGATCTCGGCAAGCAGTCTGTCCATG
TCCTCCATAGTGCCAAATTGAGGGTTGATGGCATTGTAGTCAGAAATATCGTATCC
CATATCGTCCAATGGCGAAGCATAGTGCGGAGAAAGCCAGATCACATCAGTACCA
AGGGATTTGATATGGTCGAGCTCGGAAGTGATTCCGTTTAAGTCTCCAATACCGTC
TCCGTTGGAGTCTTTGAAGGAAGCAGGCCAAACTTGGTAAACAACGGCGGATTTC
CACCAAGGTTCTTGAGACTCGATAGTCATAAGGAATTAATATTGTCAAGAGGGAT
AAAAAAATTGTGGAGGCGGAGGGTCTTTATATGGCTTGAGCTTACTAAAGACATT
CTCATCACTTTAGCGAGCGCGACGCTTTGGTATCCTCCTAACTTCCCGAAAATAAA
AAGAGGATTTCCGTTTGCGTCATGGGCTCCACCTCCTATGCACGTCCATAATAGTT
ATTTGATTGTTATCTGTACCCCAGACAAGATCCTGTTTCAGCGAGTATTAAATCAC
GGCCTAATTTATTTGAGCGAGAATTAATTTGCACATTTAATTCTCGCTGAAATAAC
TGCCAACAATTGAGTCTGGGGTACATGAATAGAAGTGACGTAATTGACCCCCACG
CTTGCTAGCCACTGCGGAGTCACTTGAACCACATGAGAGCCCGTAGGGAAGCCAG
AGCAATCAACGTCAAAGGGCTCTTAGGAAACTTGTATAACCCAAGTGTTCATTAC
GACGCTGCAGAGCTCTCACATTATATGTAGAGTGGTACGGCAGAGTCTGTAGAAC
ATACAAAGCTATCATGTCGCGTAATCTGCACAGTGCCTGCTTTCTATTGCGATACT
CCCGAAGATATCCCTATCCGCATACACGGAACTGTAGTTAATTTGCCGTGGAGTAT
CAATGTTTGTCCAACGCGCCGAAACTCTTTTGCGGGGCTCTTCTTCATCTAACTC
GTCCTAAGCTGGCTCGGGGTATCGCATAAACGTCTGCGTGCATTGCCAAGTTGTTT
TGGTCTGTGTTGAACCTTCTTTCCAAGCCCCATCCCGAACCCCGGAAAATTCCCCG

ATTATTATTTCCCCGCAAATGCGGCGTGCCAGAATCTCTCAGCCTTGCGTCATAAT

TTAGATTTCGCAATTATTTCGTGGAGCAATAGGCCGCGAGCGTCCTATTGTGCTGT

TGGCTCGAATCTCATGCACAAAAATGAGGATTTGCCAGAGTGTGTTTTCCAAGGC

GTGGCCAAAACAGAGGGTCACCAGACAACAGAGCTCACCATAATACACAGCTTTA

TGGTCTTACACTAAGTCCTCCTCCTCAACAACGCGGGGCTATACGTGTTTGCAGAA

TTCTTCCATGCGTGATTTGCAATGGTGCTTGCATACTGCAACAATGCGTATGGGGT

AATTAAAACGAGCACTCGCGAGCATTCATCAAAGGTAAAAAGCATTATTCTAAGC

TCTTTCTGGAAGCAAACTTGGGCCGCGATCACGCACCAAAATTCAGCCGGATTAA

GAATGGGCAAGCATCTCCCCACGTCATATTTGTGTGGCATTAGGACTATATATAGT

TGATATATTCTCGACACACTAGTATCCATCAGCATGCCTGAGTTCGTCGAAAACAT

CGAGAAGCCTGAAGAAGTGGAAGTCATTCCAGACATCACAAAGAAAATTAACAC

TCTTTCTGACAGTGATGATGGAAGTGGTGCTTTCAACGACTACATTGCCAGATTCG

TGGAGATTTCGACGAATGCCCAGAATAATGAGCATCAAGAAAAACACATGTCGCT

GAAGGAAGGTCTGAAAACTTTCCCGAAAGCTGCCTGCTGGTCGATTGTGCTTTCC

ACAGCCATCATCATGGAAGGATACGATACCATGCTCTTGAATAGTTTGTACTCGAT

GCAATCTTTCGCCAAAAAATACGGCCAGTACTACCCGGAAATTGACGAGTACCAG

GTCCCTGCCAAGTGGCAGACGTCGCTTTCGATGTCGACTTATGTCGGTGAAATTGT

CGGACTGTACATTGCAGGTCTTGTTGCCGAGAAATGGGGATACAGACGTACGTTG

ATATGCTTCATGGCAGCCGTTGTGGGTTTAATCTTCATTCTTTTCTTTGCGGTCGAC

GTGCAGATGCTGCTTGCCGGCGAGCTGCTCTGTGGTATTGTCTGGGGTGCATTCCA

GACTCTTACAGTGTCATACGCCTCTGAAGTCTGTCCCGTTGTCCTGAGAATCTACC

TTACCACTTATGTCAATGCGTGCTGGGTTATCGGACAGTTAATTGCTGCTTGTTTG

CTGAGAGGCACTATGACCCTGACCAACGAGTGGTCATACAAAATTCCTTTCGCCG

TTCAGTGGATCTGGCCTGTGCCAATCATGATTGGAATCTACCTGGCCCCGGAGTCG

CCCTGGTGGCTGGTTAAAAAGAACCGGGACGCAGAAGCCAAGAGAAGTATCATG

AGACTTTTGAGTCCAAACACCGAGGTGCCAGACGTTGCTCCGCTAGCCGAGGCAA

TGCTGAACAAAATGCAACTAACCATCAAGGAAGAGTCTGCGCGCACATCCAATGT

CTCGTACCTTGACTGCTTCAAACACGGCAACTTCAGAAGAACAAGGATTGCCTCC

ATGATCTGGCTCATCCAGAATATCACTGGATCTGTCTTGATGGGCTATTCGACCTA

CTTTTACATCCAGGCAGGACTTGATAGCGGTATGTCTTTCACTTTCTCTATCATCCA

ATATGCACTGGGTCTTCTTGGAACAATTGCCTCGTGGCTGCTGTCGCAAAAAATGG

GCCGTTTCGACATCTATTTCTTGGGTCTTAGCATAAACACATGCATCCTGATAATT

GTCGGAGGTTTGGGCTTCTCTTCATCGAGCAGTGCGTCTTGGGCAATTGGTTCGTT

GCTCCTTGTCTTCACTTTTGTCTACGACTCATCCATCGGTCCTATCACCTACTGTAC

GGTTGCAGAAATCCCTTCGTCAACGGTGCGTGCCAAGACAGTTGCCCTTGCAAGA

AACTGGTACAATCTGTCTCAAATCCCGCTCTCCATTGTCACTCCATACATGCTGAA

TCCGACTGCTTGGAACTGGAAAGCAAAAGCAGCCCTTTTATGGGCAGGCTTGTCT

GTTTGCTCACTGATCTACATCTGGTTCGAGTTCCCAGAAACAAAAGGAAGAACTT

ATGCTGAACTGGACATCCTATTCAAAAACGGTACCAGTGCCCGTAAGTTTAAGTC

CACTCAGGTGGAAACATTCAATCCTGAGGAAATGTTAAAAAAAATGAATAATGAG

GATATTATACAGGTTGTTGATGGCGACCTGGATGCCGGTGCGGCCACTGCTAAAG

TTTAAAAGTTGAAATGTATAGTTTCAGTTCTGCGATGTTATTATTTTACAATTGAA

TATGCTATCTCGAATGATTTTTTGTAATAGCCGTAGATGTCTTTATTATTGCATTTA

AGGGTTCAAAATTATTCATGTGAGCGTTTTATTTTACGGGACAATAAGATTTTGGG

CGAGACTTCCCCACAAATTGAATAATGTGCAATATTTTTAAGAACTAAATTTACTG

GAGACAATTTCTATGCCTCTCAGTACACGTGCAAAACCACAAACGCCTGGCCAAA

AGTCCCATTCTAAAAGGCCGTATATCAGGCCTTGTGACGCGTGCGCGTTCAAAAG

AGTACGTTGCGATGCTGAGTTCAAGTTGGATCGCAGATGCACCAACTGTTTAGTTC

ATGGAATTGAGTGTACGAATAACAGAGTCAAGCAGAGATCTGGACCCAGAAAAA

TACATAAAAAACTGAAGAGGCTATTAAAGCCTGGTAGAAGGAAGTGGCGCGT

TCAGCAATCTTCGATTAGCCACTCCGGCCCACAACATATGTGGTATTATCAATCCT

GCCACCTGCGAGAAGAGCTCAATATCGCTTAGTGAGATTCGACCGTACCTCAGAA

TTTATAAATCCCGATACTATGCACTCTGGCCAGTCCTGTCTGTTGATGACTGTCTG

AATATTCTTAATGAAAGCTCAGTTCACATAAACGATAGCGCATTTTCATGCTCAA

TGCCAACAATGCGTCGTTGTACGCTCTTTCATGCGCCTTGTGCGCTGTAATTGCAA

GACACACCACCTTCTGGTCCCTGGAAGAATTTGATACAATGGCTCATTTAGGACTC

AAAAGCTGCCCTCCGCCAGAAACTTATGTAAGGGAGGCAGAGCGAGCTATCTACA

TGTTTGACCTTGACGGGATACTCACAGAAGACCAGG

(SEQ ID NO: 3) (fragment amplified with KP-MAL1 and KP-MAL2)

TTGTCAGCTTAAAGGACTCCATTTCCTAAAATTTCAAGCAGTCCTCTCAAC

TAAATTTTTTTCCATTCCTCTGCACCCAGCCCTCTTCATCAACCGTCCAGCCTTCTC

AAAAGTCCAATGTAAGTAGCCTGCAAATTCAGGTTACAACCCCTCAATTTTCCATC

CAAGGGCGATCCTTACAAAGTTAATATCGAACAGCAGAGACTAAGCGAGTCATCA

TCACCACCCAACGATGGTGAAAAACTTTAAGCATAGATTGATGGAGGGTGTATGG

CACTTGGCGGCTGCATTAGAGTTTGAAACTATGGGGTAATACATCACATCCGGAA

CTGATCCGACTCCGAGATCATATGCAAAGCACGTGATGTACCCCGTAAACTGCTC

GGATTATCGTTGCAATTCATCGTCTTAAACAGTACAAGAAACTTTATTCATGGGTC
ATTGGACTCTGATGAGGGGCACATTTCCCCAATGATTTTTTGGGAAAGAAAGCCG
TAAGAGGACAGTTAAGCGAAAGAGACAAGACAACGAACAGCAAAAGTGACAGCT
GTCAGCTACCTAGTGGACAGTTGGGAGTTTCCAATTGGTTGGTTTTGAATTTTTAC
CCATGTTGAGTTGTCCTTGCTTCTCCTTGCAAACAATGCAAGTTGATAAGACATCA
CCTTCCAAGATAGGCTATTTTTGTCGCATAAATTTTTGTCTCGGAGTGAAAACCCC
TTTTATGTGAACAGATTACAGAAGCGTCCTACCCTTCACCGGTTGAGATGGGGAG
AAAATTAAGCGATGAGGAGACGATTATTGGTATAAAAGAAGCAACCAAAATCCC
TTATTGTCCTTTTCTGATCAGCATCAAAGAATATTGTCTTAAAACGGGCTTTTAAC
TACATTGTTCTTACACATTGCAAACCTCTTCCTTCTATTTCGGATCAACTGTATTGA
CTACATTGATCTTTTTTAACGAAGTTTACGACTTACTAAATCCCCACAAACAAATC
AACTGAGAAAAATGGTATTCCCGGAA

(SEQ ID NO: 4) (fragment amplified with KP-MAL5 and KP-MAL6)

TGGGCAGGCTTGTCTGTTTGCTCACTGATCTACATCTGGTTCGAGTTCCCA
GAAACAAAAGGAAGAACTTATGCTGAACTGGACATCCTATTCAAAAACGGTACCA
GTGCCCGTAAGTTTAAGTCCACTCAGGTGGAAACATTCAATCCTGAGGAAATGTT
AAAAAAAATGAATAATGAGGATATTATACAGGTTGTTGATGGCGACCTGGATGCC
GGTGCGGCCACTGCTAAAGTTTAAAAGTTGAAATGTATAGTTTCAGTTCTGCGATG
TTATTATTTTACAATTGAATATGCTATCTCGAATGATTTTTTGTAATAGCCGTAGAT
GTCTTTATTATTGCATTTAAGGGTTCAAAATTATTCATGTGAGCGTTTTATTTTACG
GGACAATAAGATTTTGGGCGAGACTTCCCCACAAATTGAATAATGTGCAATATTT
TTAAGAACTAAATTTACTGGAGACAATTTCTATGCCTCTCAGTACACGTGCAAAAC
CACAAACGCCTGGCCAAAAGTCCCATTCTAAAAGGCCGTATATCAGGCCTTGTGA
CGCGTGCGCGTTCAAAAGAGTACGTTGCGATGCTGAGTTCAAGTTGGATCGCAGA
TGCACCAACTGTTTAGTTCATGGAATTGAGTGTACGAATAACAGAGTCAAGCAGA
GATCTGGACCCAGAAAAATACATAAAAAAACTGAAGAGGCTATTAAAAGCCTGG
TAGAAGGAAGTGGCGCGTTCAGCAATCTTCGATTAGCCACTCCGGCCCACAACAT
ATGTGGTATTATCAATCCTGCCACCTGCGAGAAGAGCTCAATATCGCTTAGTGAG
ATTCGACCGTACCTCAGAATTTATAAATCCCGATACTATGCACTCTGGCCAGTCCT
GTCTGTTGATGACTGTCTGAATATTCTTAATGAAGCTCAGTTCACATAAACGATA
GCGCATTTTTCATGCTCAATGCCAACAATGCGTCGTTGTACGCTCTTTCATGCGCC
TTGTGCGCTGTAATTGCAAGACACACCACCTTCTGGTCCCTGGAAGAATTTGATAC
AATGGCTCATTTAGGACTCAAAAGCTGCCCTCCGCCAGAAACTTATGTAAGGGAG

GCAGAGCGAGCTATCTACATGTTTGACCTTGACGGGATACTCACAGAAGACCAGG

TTTTAACTAACTTCTTTCTGCATATATATTTTTCATCCCTTGACGAGGACAACCTGC

AGGAATTGGTCTATCTCAGACAGGCAATCAGCTGCGCACAAATGCTTCACCTTCG

AGATAACGAAGTTTTGGGAGAATCACCAAAAGATACATCACACAGATTTAAAAA

AATTTACTACTTACTTCTCATCACAGAGAGGTATGTCTCGTTCAAAAAACAACTAC

CAGTGATATTAGAGCCAACAATGCCACTTCCAAGTTTGGAAGATGATGAAGCCCC

GGATCTGCTACCTGGATTTATCGAACTTGCGCAAGTATTCAGTGTTCCTGATTGCG

CTTTCTTTAACAAGTACTCGCTCCGTGGCACGACAGCTGCACTTGATCCTTTCAGC

GAGAGCTTTGACCTAATGTTCAAAAACGAATGGATCCAAGACGTGCAAACCAAGC

TCTCTAGGTCGGTCGTTCAGGTGCGCAACCAGTCTCAAAAGGTCAATATATTAATT

TCTCGAACATGGTTGCAATCAATTGCTTGGTTAATGGCCAAAGATCGACTTCTTCT

ATCGCCTACGAGTGATCAAGCCAATTTCTTTGATCCAAGGTATCCTATTCATATCG

CGAAAGACTTTCTGATACAGACTAAAGACATGCCGTACGAGGCATTTGAAACAAA

CGGTAGTGGTGTCATCGTGAAACTAAGCGAAGTCGCCAACGCCTTGCAAACCTTC

ATCCGATTGGCTCCAGGATCGCCCGAAACAGCTTTAGCATTAGACGAGCTTGCAT

ATATCCACGGTATTATCATGCGGCAGAACTCCAACAGCTCGCTCGGGCCTCTTATA

CACCCTATCACTGAAACTTTGGAATCTCGCAGGTTCGGATTTTATCGAAATGGCTG

CGACATTTCTTATCCAAAAATAGAGTCTGCAGATTATGAAGAGAACGAAAATGAC

CATACACCCGAAATCATTGCCTCCATGCCGGAGAACTTGAATTTGACACCTCTTTT

GAGCGAGTTCGAATTTCCGAGGTGAACAAGTATTATCTGCTCACGTGTTTGGTGCA

TTTTGCAACTAGCGACAAATTTCACTGGCATATTAGTATATCATCAAATCATTAGG

ATTGCTATTTTGTGTCCACTAAGGCGCTCCAGGTGCGTGGAATGGTGCAGTATTAG

GCCCTAGCTGTCCGAGAAAAAAGGAAGCTACACCCTTTGATGTAGTGGGCTCTTT

GTGTGTTGTGAGAGGATTAAATTGGCCAGATGACACTCTTTCCAAAGATGCAAAT

AACGAGGAAGAAACATCCAGCCGGATGCCAGATGTCTGTCTGTGTTGCAAATTCG

CTCTTGATCCGGAAAAGAGTTATTGAAAAGGGCACTGTCCTGTCTCGTATTGCA

GCGTACGAGTGAACCTGAGCGTGTCAATGCAGGTTCATAAGAAGAAAATTCAGAA

GACAGATTGTGACATATTTCTCGCAAGCCATTCACTTTCCAAGATTCTAGGAGCTT

AGAGATCTTGGTGAACAGGGCTCGGGGCAAGTCCATTCCAGCTTTGTACTTCAAC

ACCATGTCGAATACGGCACCCATGCGTTCGTAGGCTAGCTCGGTGGTTGCTGGGT

CAACATAGCTTTTGATCAGAATGTAAAGAGTCTGCTATCTCCAGTAATTTCATT

GATATGTCGGGTCCGTTGCATCTAAAGGCATGATCAGGGAGGTTTTGTAGACTGC

TGAGAAGCTCATTTGCAACTTTTAGAGGAAAGTTAATCTTGAAACATTCGTGTTCG

ACCGCAATGCTCTCATCAGAACTAATTAGAAAATTCGCAGACGCAATTAACCAGC
CCAAAGACTGGAGCCAGGCTTTTGAGACAATAACATTCACTTTTTGAGCACTTGTT
GCAATTTTATCCACATCAATTCTAATTTGGAGCTTCTGTTGGAGCTCAAGGAGCCA
CAGCCTCTTCAACTCTGTGGATCCGTTCAAGGTAAAATCCTGAAACAAGTTGGTCT
CTGGAATCTGGAACATGTTGTGAGTGCGCCCTGTCTCGAGCACTTAGTATGAAGC
TCAACATAGAATTGCTTATCCGTTGCACTGAAAATTGCACACAGAGTGGTGAACC
CGTAAAGTGAATTTGAGGATTTTTCGAAATCTGCTTCAGGGAGTGGAATTGAAGG
TTCAAGAAGAACGGGCAAATTAGTTTTAAAGCTGACAAACCTTTCGGTTACCACG
AGCAAATAGTAGATTTTTTTCTGCCTATGAGCCTCAACGGGTGACAAATGAAGCA
GTGACTGTGGTTCGTGCATTTTTAAGATATCTGCAAGAGAAACAGCTTCACGGAG
GTAAATAAGACCTTTGTGCTGTTGGCCAGGCGCAATAGCATAGTATTTATGTAGG
AAGAAGGATGTCAGAAGGAGATCAATATTTGGTGTGCTTTTCAGTCTATACTCAT
AAATTATCCTCCTTGCCTCTTGAGCATATAAGCCAGCATTTTTGAGATCGCTCCTG
GCTGGAGTGTATGTGACGAAACTGTGATATTGTGCGATCGTAGCGCACAATGCGC
AACAAAGAGCATATTCTTGGGCATTGTCCTCGGCGATGGTGATACGAAAATCATC
TAAAACATTCGCTAGTTGAAATACCTGCTCCACATTCTTTAAGGGGCTTGAACGTG
TCAAAATTGAAACTGTACTTTGAATTGATACTACCGGCCAGATATCGTAAAAATTC
GAATGATATCTCAAGGTAAGGGATAATTTGTTTCAAAGAAAATGCGCACACAT
GAGCAGGTAGTGTAGACTGAGCATAGATTTCGTCGTTGAGATCCTCTGTGTTTTTG
ACTGTTCTAGTACTTTTCTTTGGACCGGATTTATGCCTCACGCGGAGGTTGGTACA
TGGAACTTTATGAGTGAGACAGTTCGAGCAAATACCACCGGTGCATGAATCTATA
TCGCACTTCACACGGCGGATGGCGCATGCGTCACAGGGTCTCGGCCGTTGTCGTTT
TTTGGTGGACGGCTGCGACATCTCGGTATTTAGATAAGCCAGCTTGTACTTGTATC
CAAAATGTGGGGCTAAAGGGGAGTGTATGATTTTAATGATATCGGTGAATGTCC
AGAAATTTGGTAAATCGCACACCAAACTTCAACGATGGACTAGCGTGCTAAACTG
ATATTCTACCAAACAACCGTTTGAGTAATTCTGACCATGACAGAGGTCTTGTGTAT
ATATTTTAAGCCCCGTCGATCGACCGCATCGCGACTCCATAAAAAAATTCATTTCA
CGCGTTATTATTATCGTATGAATATGGATCCCCTAAACGTGAAAGTGCAACAGAA
ACTTAAGGAGCTCGAAAGCCTTCAGCAGATAAGAGACTTGACGAAACACCTGAAT
GTTTCACTGGAAGAATTTGCTGGCCAAATAGAACTTCTGGGTGAGGAAGCAGGAT
GCATCGAAACCGTGACGCAAACTGGATGAGAATCATCAGAGCAGTGAGCCTAG
CCTCTAACTCTTTGTCGAACTACAAGGAAGAGGACTATGAAACGGATAGACCAAT
GACGGAGCGCCTTGTGAGATGCAAGATAGACGAGAGTCAAAAAATCATCACCAA

AAATTGACTTACTTCAATCTATCTAACCATTGTTCTAAATTGATCTTGTTGTTCAGT
TCCGACAACAGATCGTTCTCCTTCAGCAAATCGTCACGAAGGCCTTTATCAGCCTC
GAGATAATTAATATCGTCCGGATCTAACATTAAGTCCTGAACTTTAGAGGGAAGT
TCTATTTTGAGCGTCGATAACCCGGTCATCATTTTGAAGTATTCATTAGTATGCAC
GTCAGAACCATTCAAAAGCCCATCCCAATCAGTTGTCAAAAACAGCCTTTTCCAT
ATCAAGGTTTCCAAGATTTCGTGATAGTGCGAATAACCACTCAGGTTACTTATCAC
TCTTGACGGATTGGAGTGTTTGATGACAGTCAACAATTTGAACAAGTTGGCGAAA
TTGTTGTGACTGAATTCATCAAACGTCACTAATGTCAGTAGCTGGACCATCTCAAA
CAATCCCAGCTGTTGCTGCTTATCCATCTTTGCCACAATATCGCCGATCTGACTTTC
AAAATGTGTAAACTTGTTATCAACCAAGTATCTTGAGTCTTGTATGAATCCATCTT
TAGTTTTTGAATCAATTAATCTCGCATAATTCAATTGAAGCTCAATCATTTGCAAG
TGTATTTCTATCTCATCCAACAGATTGTCGTGATCCTCATTCAAAGCTATCAGTGA
AAGTTTGGCAATATTCAGTTCGAACTTTTTCTTTTCAATTTCCTCCGACTCTGCAGT
GGAAACATATGCAATCAGCTTTTCTGCTGCCTTGAAATACTCATCGTTTTCCACGT
CAAGTATCCAGGCAAATTTATAGTTGTTGATGCTTGAATTCAAAAACATGCGCAC
GTAATCTGGATATTGAGGGATGTAATTGAGGATTAGCCGAATCTTTCTGGTTCTAG
CATAGAATCCAAAAAGCACTTCTGCAAACGGTATCCATACCGCTGGAAACATTC
CTAGTTTATGTTGTATCTATG

(SEQ ID NO: 5) (fragment amplified with KP-MAL7 and KP-MAL8)

CATACCGCTGGAAACATTCCTAGTTTATGTTGTATCTATGAATATTTTTT
ACATGGTGGATTTGTTCCAATCTAGTTGTTAGTTATTTGTCGTTATAGTCACTAATA
CAGTTACCATCGGAATCTTTTCATTTCTTTTTATACGTACGTATATGTACTAGATGA
AGAATGCGACAAGGCCGACCAACAGCAATGGTGCTTGGTACCAAAGTTTGGAAG
GTGCTACCGAATTGGCCGATGATATTGAAGTGGAGTTGTCATTCTCCTCATTGGTT
GCTCCAGTAGTGGTAAAATTAACGGGGATATCTTCGGTGGAAGTGTCAATATCAC
CGTTCCAACAATAATTCTCAAAACATCTTCTACATTGCTCTGTTTGTGTTGCATTGG
ATCTCCTGCTGTCTACGAATGATTGCACATCCAACACAAGTACGCCATTCTTCA
TCAATAGTCATGTTCAAACGAGACGACACTTCAAAACCGTTTTGGATCATTCCTCT
CTTCTCGGATTCAGAGTAGTCTAACTTGAAAGTTGAAGTGTTACTCCAATACGAGA
AAGGTCTATTGGCCAGATAAACAACCAGGGGAGGAATGTGATCCGTGCCTTCAAC
AATGTCTGTCAAATTCTAGCATCACAACCAAAGAAGGTTGGCTTACTCGAAAGA
TTCAAGTTTAGGAAAGTGGTGGTATCAGGTACATAAGGGAACGTTGTTCCATTTG
CTTGAGAAGAAAACTGTCTCATGTAGGTGTTGACTAATGATGAACCGTTTGGCCA

AGAGAGGTCAGTGTCTGCACTGTTGTCAAACGCAAAGATGATATCAACGTCACGC
TCCTTTTGAAGTAGAGGCTGCAGAGGGACGTTTTGGTTATCCTCTCCACCATCAAC
CAAGTAAAGGGTGTCATTTTCCGCAATGGCACCTACTCCTGCATACGTACTTTTGT
AGAAAGGGTTGGGGGAATAGATAGCAATGTCGTCTTCGTCTTCGCTAAGTCCAGT
CAG

(SEQ ID NO: 15): Sequence of plasmid vector into which MAL promoter has been cloned

AGATCTGCTTGCCCATTCTTAATCCGGCTGAATTTTGGTGCGTGATCGCGG
CCCAAGTTTGCTTCCAGAAAGAGCTTAGAATAATGCTTTTTACCTTTGATGAATGC
TCGCGAGTGCTCGTTTTAATTACCCCATACGCATTGTTGCAGTATGCAAGCACCAT
TGCAAATCACGCATGGAAGAATTCTGCAAACACGTATAGCCCCGCGTTGTTGAGG
AGGAGGACTTAGTGTAAGACCATAAAGCTGTGTATTATGGTGAGCTCTGTTGTCT
GGTGACCCTCTGTTTTGGCCACGCCTTGGAAAACACACTCTGGCAAATCCTCATTT
TTGTGCATGAGATTCGAGCCAACAGCACAATAGGACGCTCGCGGCCTATTGCTCC
ACGAAATAATTGCGAAATCTAAATTATGACGCAAGGCTGAGAGATTCTGGCACGC
CGCATTTGCGGGGAAATAATAATCGGGGAATTTTCCGGGGTTCGGGATGGGGCTT
GGAAAGAAGGTTCAACACAGACCAAACAACTTGGCAATGCACGCAGACGTTTA
TGCGATACCCCGAGCCAGCTTAGGACGAGTTAGATGAAGAAGAGCCCCGCAAAA
AGAGTTTCGGCGCGTTGGACAAACATTGATACTCCACGGCAAATTAACTACAGTT
CCGTGTATGCGGATAGGGATATCTTCGGGAGTATCGCAATAGAAAGCAGGCACTG
TGCAGATTACGCACATGATAGCTTTGTATGTTCTACAGACTCTGCCGTACCACTC
TACATATAATGTGAGAGCTCTGCAGCGTCGTAATGAACACTTGGGTTATACAAGT
TTCCTAAGAGCCCTTTGACGTTGATTGCTCTGGCTTCCCTACGGGCTCTCATGTGG
TTCAAGTGACTCCGCAGTGGCTAGCAAGCGTGGGGGTCAATTACGTCACTTCTATT
CATGTACCCCAGACTCAATTGTTGGCAGTTATTTCAGCGAGAATTAAATGTGCAA
ATTAATTCTCGCTCAAATAAATTAGGCCGTGATTTAATACTCGCTGAAACAGGATC
TTGTCTGGGGTACAGATAACAATCAAATAACTATTATGGACGTGCATAGGAGGTG
GAGCCCATGACGCAAACGGAAATCCTCTTTTTATTTTCGGGAAGTTAGGAGGATA
CCAAAGCGTCGCGCTCGCTAAAGTGATGAGAATGTCTTTAGTAAGCTCAAGCCAT
ATAAAGACCCTCCGCCTCCACAATTTTTTATCCCTCTTGACAATATTAATTCCTTA
TGAGATTTCCTTCAATTTTTACTGCTGTTTTATTCGCAGCATCCTCCGCATTAGCTG
CTCCAGTCAACACTACAACAGAAGATGAAACGGCACAAATTCCGGCTGAAGCTGT
CATCGGTTACTCAGATTTAGAAGGGGATTTCGATGTTGCTGTTTTGCCATTTTCCA

ACAGCACAAATAACGGGTTATTGTTTATAAATACTACTATTGCCAGCATTGCTGCT
AAAGAAGAAGGGGTATCTCTCGAGAAAGAGAGGCTGAAGCTGGAGCACCAGGT
GCCCCAGGCTTGCAAGGAGCTCCTGGCCTGCAGGGTATGCCAGGAGAGAGAGGT
GCCGCTGGCTTGCCAGGTCCAAAGGGAGAGAGAGGTGATGCCGGTCCAAAGGGC
GCTGATGGAGCACCGGGTGCTCCAGGTTTGCAGGGAATGCCTGGCGAAAGAGGC
GCCGCCGGATTGCCAGGTCCAAAAGGCGAACGTGGAGATGCTGGTCCTAAGGGTG
CAGATGGAGCTCCAGGTAAAGACGGCGTTAGAGGTCTGGCAGGTCCAATTGGACC
TCCAGGTGAGAGAGGAGCTGCAGGATTGCCAGGCCCAAAGGGTGAACGTGGCGA
CGCCGGTCCAAAGGGAGCAGATGGAGCTCCGGGTAAAGATGGAGTGAGAGGACT
GGCAGGCCCTATCGGTCCACCAGGACCAGCTGGTGCCCCAGGCGCTCCTGGTTTG
CAAGGTATGCCTGGAGAGAGAGGTGCAGCTGGACTGCCAGGCCCAAAAGGTGAA
AGAGGTGATGCCGGACCAAAAGGCGCCGATGGTGCTCCAGGTAAAGATGGCGTT
AGAGGTCTTGCTGGTCCACCCGGAGCCCCTGGCTTGCAGGGCGCACCAGGATTGC
AGGGTATGCCAGGCGAGAGAGGAGCTGCAGGTCTGCCTGGTCCAAAAGGTGAAA
GAGGAGATGCTGGTCCTAAGGGTGCTGACGGCGCCCCTGGAGCTCCAGGATTGCA
GGGAATGCCAGGTGAGAGAGGCGCTGCCGGTTTGCCAGGACCTAAGGGTGAAAG
AGGTGATGCTGGCCCAAAAGGTGCCGATGGAGCTCCAGGAAAAGATGGTGTTAG
AGGACTGGCCGGTCCTATCGGCCCTCCTGGAGAAAGAGGAGCTGCTGGTTTGCCT
GGCCCAAAAGGCGAGAGAGGTGACGCTGGCCCAAAAGGAGCCGACGGTGCTCCT
GGCAAGGACGGAGTGAGAGGCCTGGCCGGTCCTATTGGTCCACCAGGACCTGCTG
GCGCTCCAGGAGCTCCTGGTTTGCAGGGTATGCCAGGTGAAAGAGGAGCCGCAGG
CCTGCCAGGTCCTAAGGGTGAGAGAGGTGACGCCGGTCCTAAAGGCGCTGATGGA
GCTCCTGGTAAAGATGGCGTTAGAGGTTTGGCCGGACCCCCAGGTGCCCCAGGTC
TGCAAGGAGCTCCAGGTCTTCAGGGTATGCCAGGCGAAAGAGGAGCTGCTGGTTT
GCCAGGCCCAAAGGGTGAAAGAGGAGATGCCGGCCCAAAAGGAGCTGACGGTGC
ACCAGGCGCTCCAGGCCTGCAGGGTATGCCAGGAGAAAGAGGTGCTGCTGGTTTG
CCAGGACCAAAGGGAGAAAGAGGCGACGCCGGTCCAAAGGGTGCAGATGGAGCT
CCTGGCAAAGACGGAGTGAGAGGCTTGGCAGGTCCAATCGGACCGCCAGGCGAG
AGAGGTGCTGCCGGCTTGCCAGGTCCAAAGGGTGAAAGAGGAGACGCAGGTCCT
AAAGGTGCTGACGGCGCACCAGGAAAAGATGGTGTTAGAGGTCTGGCTGGACCT
ATCGGCCCACCAGGACCAGCTGGTGCTCCAGGTGCTCCTGGCCTTCAGGGCATGC
CAGGAGAAAGAGGTGCTGCCGGTCTGCCTGGACCTAAGGGCGAAAGAGGCGATG
CAGGTCCAAAGGGTGCTGATGGAGCTCCTGGTAAAGATGGTGTTAGAGGATTGGC

AGGCCCACCCGGGTAATAGTCTAGAGTCGACTGAGTTTGTAGCCTTAGACATGAC

TGTTCCTCAGTTCAAGTTGGGCACTTACGAGAAGACCGGTCTTGCTAGATTCTAAT

CAAGAGGATGTCAGAATGCCATTTGCCTGAGAGATGCAGGCTTCATTTTTGATACT

TTTTTATTTGTAACCTATATAGTATAGGATTTTTTTTGTCATTTTGTTTCTTCTCGTA

CGAGCTTGCTCCTGATCAGCCTATCTCGCAGCTGATGAATATCTTGTGGTAGGGGT

TTGGGAAAATCATTCGAGTTTGATGTTTTTCTTGGTATTTCCCACTCCTCTTCAGAG

TACAGAAGATTAAGTGAGACCTTCGTTTGTGCACTAGTCCCACACACCATAGCTTC

AAAATGTTTCTACTCCTTTTTTACTCTTCCAGATTTTCTCGGACTCCGCGCATCGCC

GTACCACTTCAAAACACCCAAGCACAGCATACTAAATTTTCCCTCTTTCTTCCTCT

AGGGTGTCGTTAATTACCCGTACTAAAGGTTTGGAAAAGAAAAAAGAGACCGCCT

CGTTTCTTTTTCTTCGTCGAAAAAGGCAATAAAAATTTTATCACGTTTCTTTTTCT

TGAAATTTTTTTTTTAGTTTTTTTCTCTTTCAGTGACCTCCATTGATATTTAAGTTA

ATAAACGGTCTTCAATTTCTCAAGTTTCAGTTTCATTTTTCTTGTTCTATTACAACT

TTTTTACTTCTTGTTCATTAGAAAGAAAGCATAGCAATCTAATCTAAGGGGCGGT

GTTGACAATTAATCATCGGCATAGTATATCGGCATAGTATAATACGACAAGGTGA

GGAACTAAACCATGGCCAAGTTGACCAGTGCCGTTCCGGTGCTCACCGCGCGCGA

CGTCGCCGGAGCGGTCGAGTTCTGGACCGACCGGCTCGGGTTCTCCCGCGACTTC

GTGGAGGACGACTTCGCCGGTGTGGTCCGGGACGACGTGACCTGTTCATCAGCG

CGGTCCAGGACCAGGTGGTGCCGGACAACACCCTGGCCTGGGTGTGGGTGCGCGG

CCTGGACGAGCTGTACGCCGAGTGGTCGGAGGTCGTGTCCACGAACTTCCGGGAC

GCCTCCGGGCCGGCCATGACCGAGATCGGCGAGCAGCCGTGGGGGCGGGAGTTC

GCCCTGCGCGACCCGGCCGGCAACTGCGTGCACTTCGTCGCCGAGGAGCAGGACT

GACACGTCCGACGGCGGCCCACGGGTCCCAGGCCTCGGAGATCCGTCCCCCTTTT

CCTTTGTCGATATCATGTAATTAGTTATGTCACGCTTACATTCACGCCCTCCCCCCA

CATCCGCTCTAACCGAAAAGGAAGGAGTTAGACAACCTGAAGTCTAGGTCCCTAT

TTATTTTTTTATAGTTATGTTAGTATTAAGAACGTTATTTATATTTCAAATTTTTCTT

TTTTTCTGTACAGACGCGTGTACGCATGTAACATTATACTGAAAACCTTGCTTGA

GAAGGTTTTGGGACGCTCGAAGGCTTTAATTTGCAAGCTGGAGACCAACATGTGA

GCAAAAGGCCAGCAAAAGGCCAGGAACCGTAAAAAGGCCGCGTTGCTGGCGTTT

TTCCATAGGCTCCGCCCCCCTGACGAGCATCACAAAAATCGACGCTCAAGTCAGA

GGTGGCGAAACCCGACAGGACTATAAAGATACCAGGCGTTTCCCCCTGGAAGCTC

CCTCGTGCGCTCTCCTGTTCCGACCCTGCCGCTTACCGGATACCTGTCCGCCTTTCT

CCCTTCGGGAAGCGTGGCGCTTTCTCATAGCTCACGCTGTAGGTATCTCAGTTCGG

TGTAGGTCGTTCGCTCCAAGCTGGGCTGTGTGCACGAACCCCCGTTCAGCCCGAC
CGCTGCGCCTTATCCGGTAACTATCGTCTTGAGTCCAACCCGGTAAGACACGACTT
ATCGCCACTGGCAGCAGCCACTGGTAACAGGATTAGCAGAGCGAGGTATGTAGGC
GGTGCTACAGAGTTCTTGAAGTGGTGGCCTAACTACGGCTACACTAGAAGAACAG
TATTTGGTATCTGCGCTCTGCTGAAGCCAGTTACCTTCGGAAAAAGAGTTGGTAGC
TCTTGATCCGGCAAACAAACCACCGCTGGTAGCGGTGGTTTTTTTGTTTGCAAGCA
GCAGATTACGCGCAGAAAAAAAGGATCTCAAGAAGATCCTTTGATCTTTTCTACG
GGGTCTGACGCTCAGTGGAACGAAAACTCACGTTAAGGGATTTTGGTCATGAGAT
C

(SEQ ID NO: 16): P-mal: sucrose-inducible MAL promoter (included in MAL cluster)

GCTTGCCCATTCTTAATCCGGCTGAATTTTGGTGCGTGATCGCGGCCCAAG
TTTGCTTCCAGAAAGAGCTTAGAATAATGCTTTTTACCTTTGATGAATGCTCGCGA
GTGCTCGTTTTAATTACCCCATACGCATTGTTGCAGTATGCAAGCACCATTGCAAA
TCACGCATGGAAGAATTCTGCAAACACGTATAGCCCCGCGTTGTTGAGGAGGAGG
ACTTAGTGTAAGACCATAAAGCTGTGTATTATGGTGAGCTCTGTTGTCTGGTGACC
CTCTGTTTTGGCCACGCCTTGGAAAACACTCTGGCAAATCCTCATTTTTGTGCA
TGAGATTCGAGCCAACAGCACAATAGGACGCTCGCGGCCTATTGCTCCACGAAAT
AATTGCGAAATCTAAATTATGACGCAAGGCTGAGAGATTCTGGCACGCCGCATTT
GCGGGGAAATAATAATCGGGGAATTTTCCGGGGTTCGGGATGGGGCTTGGAAAG
AAGGTTCAACACAGACCAAAACAACTTGGCAATGCACGCAGACGTTTATGCGATA
CCCCGAGCCAGCTTAGGACGAGTTAGATGAAGAAGAGCCCCGCAAAAAGAGTTT
CGGCGCGTTGGACAAACATTGATACTCCACGGCAAATTAACTACAGTTCCGTGTA
TGCGGATAGGGATATCTTCGGGAGTATCGCAATAGAAAGCAGGCACTGTGCAGAT
TACGCGACATGATAGCTTTGTATGTTCTACAGACTCTGCCGTACCACTCTACATAT
AATGTGAGAGCTCTGCAGCGTCGTAATGAACACTTGGGTTATACAAGTTTCCTAA
GAGCCCTTTGACGTTGATTGCTCTGGCTTCCCTACGGGCTCTCATGTGGTTCAAGT
GACTCCGCAGTGGCTAGCAAGCGTGGGGGTCAATTACGTCACTTCTATTCATGTAC
CCCAGACTCAATTGTTGGCAGTTATTTCAGCGAGAATTAAATGTGCAAATTAATTC
TCGCTCAAATAAATTAGGCCGTGATTTAATACTCGCTGAAACAGGATCTTGTCTGG
GGTACAGATAACAATCAAATAACTATTATGGACGTGCATAGGAGGTGGAGCCCAT
GACGCAAACGGAAATCCTCTTTTATTTTCGGGAAGTTAGGAGGATACCAAAGCG
TCGCGCTCGCTAAAGTGATGAGAATGTCTTTAGTAAGCTCAAGCCATATAAAGAC
CCTCCGCCTCCACAATTTTTTTATCCTCTTGACAATATTAATTCCTT

(SEQ ID NO: 17): MF-a: signal sequence of target protein

ATGAGATTTCCTTCAATTTTTACTGCTGTTTTATTCGCAGCATCCTCCGCAT
TAGCTGCTCCAGTCAACACTACAACAGAAGATGAAACGGCACAAATTCCGGCTGA
AGCTGTCATCGGTTACTCAGATTAGAAGGGGATTTCGATGTTGCTGTTTTGCCAT
TTTCCAACAGCACAAATAACGGGTTATTGTTTATAAATACTACTATTGCCAGCATT
GCTGCTAAGAAGAAGGGGTATCTCTCGAGAAAGAGAGGCTGAAGCT

(SEQ ID NO: 18): NRC3: target protein (CBE3)

GGAGCACCAGGTGCCCCAGGCTTGCAAGGAGCTCCTGGCCTGCAGGGTAT
GCCAGGAGAGAGAGGTGCCGCTGGCTTGCCAGGTCCAAAGGGAGAGAGAGGTGA
TGCCGGTCCAAAGGGCGCTGATGGAGCACCGGGTGCTCCAGGTTTGCAGGGAATG
CCTGGCGAAAGAGGCGCCGCCGGATTGCCAGGTCCAAAAGGCGAACGTGGAGAT
GCTGGTCCTAAGGGTGCAGATGGAGCTCCAGGTAAAGACGGCGTTAGAGGTCTGG
CAGGTCCAATTGGACCTCCAGGTGAGAGAGGAGCTGCAGGATTGCCAGGCCCAA
AGGGTGAACGTGGCGACGCCGGTCCAAAGGGAGCAGATGGAGCTCCGGGTAAAG
ATGGAGTGAGAGGACTGGCAGGCCCTATCGGTCCACCAGGACCAGCTGGTGCCCC
AGGCGCTCCTGGTTTGCAAGGTATGCCTGGAGAGAGAGGTGCAGCTGGACTGCCA
GGCCCAAAAGGTGAAAGAGGTGATGCCGGACCAAAAGGCGCCGATGGTGCTCCA
GGTAAAGATGGCGTTAGAGGTCTTGCTGGTCCACCCGGAGCCCCTGGCTTGCAGG
GCGCACCAGGATTGCAGGGTATGCCAGGCGAGAGAGGAGCTGCAGGTCTGCCTG
GTCCAAAAGGTGAAAGAGGAGATGCTGGTCCTAAGGGTGCTGACGGCGCCCCTG
GAGCTCCAGGATTGCAGGGAATGCCAGGTGAGAGAGGCGCTGCCGGTTTGCCAG
GACCTAAGGGTGAAAGAGGTGATGCTGGCCCAAAAGGTGCCGATGGAGCTCCAG
GAAAAGATGGTGTTAGAGGACTGGCCGGTCCTATCGGCCCTCCTGGAGAAAGAGG
AGCTGCTGGTTTGCCTGGCCCAAAAGGCGAGAGAGGTGACGCTGGCCCAAAAGG
AGCCGACGGTGCTCCTGGCAAGGACGGAGTGAGAGGCCTGGCCGGTCCTATTGGT
CCACCAGGACCTGCTGGCGCTCCAGGAGCTCCTGGTTTGCAGGGTATGCCAGGTG
AAAGAGGAGCCGCAGGCCTGCCAGGTCCTAAGGGTGAGAGAGGTGACGCCGGTC
CTAAAGGCGCTGATGGAGCTCCTGGTAAAGATGGCGTTAGAGGTTTGGCCGGACC
CCCAGGTGCCCCAGGTCTGCAAGGAGCTCCAGGTCTTCAGGGTATGCCAGGCGAA
AGAGGAGCTGCTGGTTTGCCAGGCCCAAAGGGTGAAAGAGGAGATGCCGGCCCA
AAAGGAGCTGACGGTGCACCAGGCGCTCCAGGCCTGCAGGGTATGCCAGGAGAA
AGAGGTGCTGCTGGTTTGCCAGGACCAAAGGGAGAAAGAGGCGACGCCGGTCCA
AAGGGTGCAGATGGAGCTCCTGGCAAAGACGGAGTGAGAGGCTTGGCAGGTCCA

ATCGGACCGCCAGGCGAGAGAGGTGCTGCCGGCTTGCCAGGTCCAAAGGGTGAA
AGAGGAGACGCAGGTCCTAAAGGTGCTGACGGCGCACCAGGAAAAGATGGTGTT
AGAGGTCTGGCTGGACCTATCGGCCCACCAGGACCAGCTGGTGCTCCAGGTGCTC
CTGGCCTTCAGGGCATGCCAGGAGAAAGAGGTGCTGCCGGTCTGCCTGGACCTAA
GGGCGAAAGAGGCGATGCAGGTCCAAAGGGTGCTGATGGAGCTCCTGGTAAAGA
TGGTGTTAGAGGATTGGCAGGCCCACCCGGG

(SEQ ID NO: 19): pPICZα-CBE3

AGATCTAACATCCAAAGACGAAAGGTTGAATGAAACCTTTTTGCCATCCG
ACATCCACAGGTCCATTCTCACACATAAGTGCCAAACGCAACAGGAGGGGATACA
CTAGCAGCAGACCGTTGCAAACGCAGGACCTCCACTCCTCTTCCTCAACACCCA
CTTTTGCCATCGAAAAACCAGCCCAGTTATTGGGCTTGATTGGAGCTCGCTCATTC
CAATTCCTTCTATTAGGCTACTAACACCATGACTTTATTAGCCTGTCTATCCTGGCC
CCCCTGGCGAGGTTCATGTTTGTTTATTTCCGAATGCAACAAGCTCCGCATTACAC
CCGAACATCACTCCAGATGAGGGCTTTCTGAGTGTGGGGTCAAATAGTTTCATGTT
CCCCAAATGGCCCAAAACTGACAGTTTAAACGCTGTCTTGGAACCTAATATGACA
AAAGCGTGATCTCATCCAAGATGAACTAAGTTTGGTTCGTTGAAATGCTAACGGC
CAGTTGGTCAAAAGAAACTTCCAAAGTCGGCATACCGTTTGTCTTGTTTGGTAT
TGATTGACGAATGCTCAAAAATAATCTCATTAATGCTTAGCGCAGTCTCTCTATCG
CTTCTGAACCCCGGTGCACCTGTGCCGAAACGCAAATGGGGAAACACCCGCTTTT
TGGATGATTATGCATTGTCTCCACATTGTATGCTTCCAAGATTCTGGTGGGAATAC
TGCTGATAGCCTAACGTTCATGATCAAAATTTAACTGTTCTAACCCCTACTTGACA
GCAATATATAAACAGAAGGAAGCTGCCCTGTCTTAAACCTTTTTTTTTATCATCAT
TATTAGCTTACTTTCATAATTGCGACTGGTTCCAATTGACAAGCTTTTGATTTTAAC
GACTTTTAACGACAACTTGAGAAGATCAAAAAACAACTAATTATTCGAAGAATCC
AAACGATGAGATTTCCTTCAATTTTTACTGCAGTTTTATTCGCAGCATCCTCCGCA
TTAGCTGCTCCAGTCAACACTACAACAGAAGATGAAACGGCACAAATTCCGGCTG
AAGCTGTCATCGGTTACTCAGATTTAGAAGGGGATTTCGATGTTGCTGTTTTGCCA
TTTTCCAACAGCACAAATAACGGGTTATTGTTTATAAATACTACTATTGCCAGCAT
TGCTGCTAAAGAAGAAGGGGTATCTCTCGAGAAAGAGAGGCTGAAGCTGGAGC
TCCAGGTGCTCCAGGCCTTCAGGGTGCTCCAGGTTTGCAGGGTATGCCAGGAGAG
AGAGGAGCTGCCGGTTTGCCTGGCCCAAAGGGTGAGAGAGGTGACGCTGGTCCTA
AAGGTGCTGACGGAGCTCCAGGAGCTCCTGGACTGCAAGGCATGCCTGGTGAAAG
AGGTGCAGCTGGCCTGCCAGGACCAAAAGGAGAAAGAGGCGATGCAGGCCCAAA

GGGAGCAGATGGTGCCCCAGGTAAAGACGGTGTTAGAGGCTTGGCTGGACCTATC
GGACCTCCAGGTGAAAGAGGTGCAGCTGGCCTGCCAGGACCAAAAGGAGAAAGA
GGCGATGCAGGCCCAAAGGGAGCAGATGGTGCCCCAGGTAAAGACGGTGTTAGA
GGCTTGGCTGGACCTATCGGACCACCTGGTCCAGCTGGAGCCCCTGGTGCTCCAG
GTTTGCAGGGTATGCCAGGAGAGAGAGGAGCTGCCGGTTTGCCTGGTCCAAAGGG
CGAAAGAGGTGATGCCGGACCGAAAGGCGCTGATGGCGCCCCAGGCAAGGATGG
CGTGAGAGGTCTGGCCGGTCCACCCGGTGCTCCAGGCCTTCAGGGTGCTCCAGGT
TTGCAGGGTATGCCAGGAGAGAGAGGAGCTGCCGGTTTGCCTGGCCCAAAGGGTG
AGAGAGGTGACGCTGGTCCTAAAGGTGCTGACGGAGCTCCAGGAGCTCCTGGACT
GCAAGGCATGCCTGGTGAAAGAGGTGCAGCTGGCCTGCCAGGACCAAAAGGAGA
AAGAGGCGATGCAGGCCCAAAGGGAGCAGATGGTGCCCCAGGTAAAGACGGTGT
TAGAGGCTTGGCTGGACCTATCGGACCTCCAGGTGAAAGAGGTGCAGCTGGCCTG
CCAGGACCAAAAGGAGAAAGAGGCGATGCAGGCCCAAAGGGAGCAGATGGTGCC
CCAGGTAAAGACGGTGTTAGAGGCTTGGCTGGACCTATCGGACCACCTGGTCCAG
CTGGAGCCCCTGGTGCTCCAGGTTTGCAGGGTATGCCAGGAGAGAGAGGAGCTGC
CGGTTTGCCTGGTCCAAAGGGCGAAAGAGGTGATGCCGGACCGAAAGGCGCTGA
TGGCGCCCCAGGCAAGGATGGCGTGAGAGGTCTGGCCGGTCCACCCGGTGCTCCA
GGCCTTCAGGGTGCTCCAGGTTTGCAGGGTATGCCAGGAGAGAGAGGAGCTGCCG
GTTTGCCTGGCCCAAAGGGTGAGAGAGGTGACGCTGGTCCTAAAGGTGCTGACGG
AGCTCCAGGAGCTCCTGGACTGCAAGGCATGCCTGGTGAAAGAGGTGCAGCTGGC
CTGCCAGGACCAAAAGGAGAAAGAGGCGATGCAGGCCCAAAGGGAGCAGATGGT
GCCCCAGGTAAAGACGGTGTTAGAGGCTTGGCTGGACCTATCGGACCTCCAGGTG
AAAGAGGTGCAGCTGGCCTGCCAGGACCAAAAGGAGAAAGAGGCGATGCAGGCC
CAAAGGGAGCAGATGGTGCCCCAGGTAAAGACGGTGTTAGAGGCTTGGCTGGAC
CTATCGGACCACCTGGTCCAGCTGGAGCCCCTGGTGCTCCAGGTTTGCAGGGTAT
GCCAGGAGAGAGAGGAGCTGCCGGTTTGCCTGGTCCAAAGGGCGAAAGAGGTGA
TGCCGGACCGAAAGGCGCTGATGGCGCCCCAGGCAAGGATGGCGTGAGAGGTCT
GGCCGGTCCACCCGGGTAAGCGGCCGCCAGCTTTCTAGAACAAAAACTCATCTCA
GAAGAGGATCTGAATAGCGCCGTCGACCATCATCATCATCATCATTGAGTTTGTA
GCCTTAGACATGACTGTTCCTCAGTTCAAGTTGGGCACTTACGAGAAGACCGGTCT
TGCTAGATTCTAATCAAGAGGATGTCAGAATGCCATTTGCCTGAGAGATGCAGGC
TTCATTTTTGATACTTTTTTATTTGTAACCTATATAGTATAGGATTTTTTTTGTCATT
TTGTTTCTTCTCGTACGAGCTTGCTCCTGATCAGCCTATCTCGCAGCTGATGAATAT

CTTGTGGTAGGGGTTTGGGAAAATCATTCGAGTTTGATGTTTTTCTTGGTATTTCCC

ACTCCTCTTCAGAGTACAGAAGATTAAGTGAGACCTTCGTTTGTGCGGATCCCCCA

CACACCATAGCTTCAAAATGTTTCTACTCCTTTTTTACTCTTCCAGATTTTCTCGGA

CTCCGCGCATCGCCGTACCACTTCAAAACACCCAAGCACAGCATACTAAATTTCC

CCTCTTTCTTCCTCTAGGGTGTCGTTAATTACCCGTACTAAAGGTTTGGAAAAGAA

AAAAGAGACCGCCTCGTTTCTTTTTCTTCGTCGAAAAAGGCAATAAAAATTTTTAT

CACGTTTCTTTTTCTTGAAAATTTTTTTTTTGATTTTTTTCTCTTTCGATGACCTCC

CATTGATATTTAAGTTAATAAACGGTCTTCAATTTCTCAAGTTTCAGTTTCATTTTT

CTTGTTCTATTACAACTTTTTTTACTTCTTGCTCATTAGAAAGAAAGCATAGCAATC

TAATCTAAGGGCGGTGTTGACAATTAATCATCGGCATAGTATATCGGCATAGTAT

AATACGACAAGGTGAGGAACTAAACCATGGCCAAGTTGACCAGTGCCGTTCCGGT

GCTCACCGCGCGCGACGTCGCCGGAGCGGTCGAGTTCTGGACCGACCGGCTCGGG

TTCTCCCGGGACTTCGTGGAGGACGACTTCGCCGGTGTGGTCCGGGACGACGTGA

CCCTGTTCATCAGCGCGGTCCAGGACCAGGTGGTGCCGGACAACACCCTGGCCTG

GGTGTGGGTGCGCGGCCTGGACGAGCTGTACGCCGAGTGGTCGGAGGTCGTGTCC

ACGAACTTCCGGGACGCCTCCGGGCCGGCCATGACCGAGATCGGCGAGCAGCCGT

GGGGGCGGGAGTTCGCCCTGCGCGACCCGGCCGGCAACTGCGTGCACTTCGTGGC

CGAGGAGCAGGACTGACACGTCCGACGCGGCCCGACGGGTCCGAGGCCTCGGAG

ATCCGTCCCCCTTTTCCTTTGTCGATATCATGTAATTAGTTATGTCACGCTTACATT

CACGCCCTCCCCCCACATCCGCTCTAACCGAAAAGGAAGGAGTTAGACAACCTGA

AGTCTAGGTCCCTATTTATTTTTTTATAGTTATGTTAGTATTAAGAACGTTATTTAT

ATTTCAAATTTTTCTTTTTTTTCTGTACAGACGCGTGTACGCATGTAACATTATACT

GAAAACCTTGCTTGAGAAGGTTTTGGGACGCTCGAAGGCTTTAATTTGCAAGCTG

GAGACCAACATGTGAGCAAAAGGCCAGCAAAAGGCCAGGAACCGTAAAAAGGCC

GCGTTGCTGGCGTTTTTCCATAGGCTCCGCCCCCCTGACGAGCATCACAAAAATCG

ACGCTCAAGTCAGAGGTGGCGAAACCCGACAGGACTATAAAGATACCAGGCGTTT

CCCCCTGGAAGCTCCCTCGTGCGCTCTCCTGTTCCGACCCTGCCGCTTACCGGATA

CCTGTCCGCCTTTCTCCCTTCGGGAAGCGTGGCGCTTTCTCATAGCTCACGCTGTA

GGTATCTCAGTTCGGTGTAGGTCGTTCGCTCCAAGCTGGGCTGTGTGCACGAACCC

CCCGTTCAGCCCGACCGCTGCGCCTTATCCGGTAACTATCGTCTTGAGTCCAACCC

GGTAAGACACGACTTATCGCCACTGGCAGCAGCCACTGGTAACAGGATTAGCAGA

GCGAGGTATGTAGGCGGTGCTACAGAGTTCTTGAAGTGGTGGCCTAACTACGGCT

ACACTAGAAGAACAGTATTTGGTATCTGCGCTCTGCTGAAGCCAGTTACCTTCGG

AAAAAGAGTTGGTAGCTCTTGATCCGGCAAACAAACCACCGCTGGTAGCGGTGGT
TTTTTGTTTGCAAGCAGCAGATTACGCGCAGAAAAAAGGATCTCAAGAAGATC
CTTTGATCTTTTCTACGGGGTCTGACGCTCAGTGGAACGAAAACTCACGTTAAGGG
ATTTTGGTCATGAGATC

[Sequence list] International application 23F00372W1JP24020485_2.xml based on International Patent Cooperation Treaty

**Claims**

1. A recombinant yeast comprising:
a first exogenous sucrose-inducible promoter for expressing a target protein.

2. The recombinant yeast according to claim 1, further comprising:
an exogenous gene encoding the target protein.

3. The recombinant yeast according to claim 1, further comprising:
an exogenous gene that confers sucrose assimilation.

4. The recombinant yeast according to claim 3,
wherein the exogenous gene that confers sucrose assimilation is linked to a second exogenous sucrose-inducible promoter.

5. The recombinant yeast according to claim 3,
wherein the exogenous gene that confers sucrose assimilation is a maltase gene.

6. The recombinant yeast according to claim 3,
wherein the first exogenous sucrose-inducible promoter and the exogenous gene that confers sucrose assimilation are derived from a yeast having an $\alpha$-glucosidase which has sucrose-hydrolyzing activity.

7. The recombinant yeast according to claim 6,
wherein the yeast having an $\alpha$-glucosidase which has sucrose-hydrolyzing activity belongs to any of the genus Ogataea, Candida, Cyberlindnera, Wickerhamomyces, Clavispora, Debaryomyces, Meyerozyma, Scheffersomyces, Metschnikowia, Spathaspora, Babjeviella, Hypopichia, or Yamadazyma.

8. The recombinant yeast according to claim 7,
wherein the yeast having an $\alpha$-glucosidase which has sucrose-hydrolyzing activity is Ogataea parapolymorpha.

9. The recombinant yeast according to any one of claims 1 to 8,
wherein the recombinant yeast is the genus Komagataella.

10. A method for producing a target protein, comprising:
culturing the recombinant yeast according to any one of claims 1 to 8 in a culture medium containing sucrose as a carbon source.

11. The method for producing a target protein according to claim 10,
wherein the target protein is an antibody, a bioactive protein, a bioactive polypeptide, an extracellular matrix, an artificial protein, or an enzyme.

12. The method for producing a target protein according to claim 10,
wherein a concentration of the sucrose in the culture medium during the culture is 0.5 to 2 g/mL.

13. The method for producing a target protein according to claim 10,
wherein an addition rate of the sucrose is 2 to 100 g/hour per 0.9 L of the culture medium at the start of the culture.

**14.** A kit for producing the recombinant yeast according to any one of claims 1 to 8, the kit comprising:

a yeast; and
an expression construct containing a sucrose-inducible promoter.

**15.** The kit according to claim 14,

wherein the expression construct contains an exogenous gene encoding the target protein, and
the sucrose-inducible promoter induces expression of the exogenous gene encoding the target protein.

**16.** A method for producing a recombinant yeast, which is a method for producing the recombinant yeast according to any one of claims 1 to 8, the method comprising:
introducing an expression construct containing a sucrose-inducible promoter into a yeast.

**17.** The method for producing a recombinant yeast according to claim 16,
wherein the expression construct containing the sucrose-inducible promoter is introduced into a genome of the yeast.

**18.** The method for producing a recombinant yeast according to claim 16,

wherein the expression construct containing the sucrose-inducible promoter contains an exogenous gene encoding the sucrose-inducible promoter and the target protein, and
the sucrose-inducible promoter is an expression construct that induces expression of an exogenous gene encoding the target protein.

**19.** The method for producing a recombinant yeast according to claim 16,
wherein the expression construct containing the sucrose-inducible promoter is introduced into a genome of a yeast having an exogenous gene that confers sucrose assimilation.

EP 4 726 051 A1

# FIG. 1

KP-MAL1
KP-MAL3
KP-MAL2
KP-MAL5
KP-MAL4
KP-MAL7
KP-MAL6
KP-MAL8

MAL CLUSTER

AOX2'    AG1        AGT1      MAL        MAL        'AOX2
                              -ACT       -ACT

# FIG. 2

KP-MAL3        KP-MAL4

PARTIAL REGION
OF AOX2
PORTION OF 5' REGION OF
MAL CLUSTER

Product A

KP-MAL5        KP-MAL6

PORTION OF 3' REGION OF
MAL CLUSTER
PARTIAL REGION
OF AOX2

Product B

# FIG. 3

# FIG. 4

FIG. 5

EP 4 726 051 A1

## FIG. 6

(1) BgI II
AleI – MslI (76)
Bmrl (178)
PflMI (190)
Dral – Pmel (413)
BlpI (588)
NsiI (677)
BstXI – XcmI (706)
MfeI (864)
HindIII (872)
BstBI (933)
PstI (975)
PsiI (1144)
BsrDI (1163)
PaeR7I – XhoI (1192)

(4956) AlwNI
(4848) PspFI
(4844) BseYI
(4713) BssSI – BssSαI
(4540) PciI
(4451) MluI
(4444) BsrGI
CYC1 terminator
(4277) EcoRV
(4115) FseI
(3953) SgrAI
(3885) Aat II
(3883) ZraI
(3875) BssHII – MauBI
(3842) MscI
(3837) NcoI – StyI
EM7 promoter
TEF1 promoter
(3352) BamHI
AOX1 terminator
(3203) BsiWI
(3071) AgeI
(2990) AccI
(2989) SalI
(2946) XbaI
(2933) NotI

ColE1 origin
AOX1 promoter
Zeo-R
MFa-CBE3

pPICZ-CBE3
5269 bp

EP 4 726 051 A1

# EP 4 726 051 A1

## INTERNATIONAL SEARCH REPORT

| International application No. |
|---|
| **PCT/JP2024/020485** |

| A. | CLASSIFICATION OF SUBJECT MATTER |
|---|---|

*C12P 21/00*(2006.01)i; *C12N 1/19*(2006.01)i; *C12N 15/81*(2006.01)i
FI:    C12P21/00 C; C12N1/19 ZNA; C12N15/81 Z

According to International Patent Classification (IPC) or to both national classification and IPC

| B. | FIELDS SEARCHED |
|---|---|

Minimum documentation searched (classification system followed by classification symbols)

C12P21/00; C12N1/19; C12N15/81

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | BOONCHOO, Kriengsak et al. Sucrose-inducible heterologous expression of phytase in high cell density cultivation of the thermotolerant methylotrophic yeast Ogataea thermomethanolic a. FEMS Microbiology Letters. 2019, vol. 366, no. 5, fnz052 pp. 1-7, doi.org/10.1093/femsle/fnz052<br>abstract, p. 2, left column, lines 22-34, materials and methods, results and discussion, conclusion, fig. 1-5 | 1-19 |
| X | PUSEENAM, Aekkachai et al. A novel sucrose-based expression system for heterologous proteins expression in thermotolerant methylotrophic yeast Ogataea thermomethanolica. FEMS Microbiology Letters. 2018, vol. 365, no. 20, fny238 pp. 1-6, doi: 10.1093/femsle/fny238<br>abstract, materials and methods, results and discussion, conclusion, table 1, fig. 1-4 | 1-19 |
| A | JP 2018-515098 A (IMPOSSIBLE FOODS INC.) 14 June 2018 (2018-06-14)<br>claims, examples | 1-19 |

☑ Further documents are listed in the continuation of Box C.       ☑ See patent family annex.

| | |
|---|---|
| *     Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **09 July 2024** | **23 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

40

# EP 4 726 051 A1

**INTERNATIONAL SEARCH REPORT**

International application No.

**PCT/JP2024/020485**

## C. DOCUMENTS CONSIDERED TO BE RELEVANT

| Category* | Citation of document, with indication, where appropriate, of the relevant passages | Relevant to claim No. |
|---|---|---|
| A | JP 2014-522663 A (FUJIFILM DIOSYNTH BIOTECHNOLOGIES UK LTD.) 08 September 2014 (2014-09-08)<br>claims | 1-19 |
| A | JP 2007-89512 A (KABUSHIKI KAISHA TOYOTA CHUO KENKYUSHO) 12 April 2007 (2007-04-12)<br>claims | 1-19 |
| A | JP 2018-522565 A (LONZA LTD.) 16 August 2018 (2018-08-16)<br>claims | 1-19 |
| A | JP 2019-505194 A (BISY E.U) 28 February 2019 (2019-02-28)<br>claims | 1-19 |
| P, X | WO 2023/219575 A1 (NATIONAL SCIENCE AND TECHNOLOGY DEVELOPMENT AGENCY) 16 November 2023 (2023-11-16)<br>claims, examples | 1-19 |

Form PCT/ISA/210 (second sheet) (July 2022)

41

## INTERNATIONAL SEARCH REPORT

| International application No. |
| --- |
| **PCT/JP2024/020485** |

| **Box No. I**  **Nucleotide and/or amino acid sequence(s) (Continuation of item 1.c of the first sheet)** |
| --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/020485**

| Patent document cited in search report | | | Publication date (day/month/year) | Patent family member(s) | | | Publication date (day/month/year) |
|---|---|---|---|---|---|---|---|
| JP | 2018-515098 | A | 14 June 2018 | US | 2017/0349637 | A1 | |
| | | | | claims, examples | | | |
| | | | | WO | 2016/183163 | A1 | |
| | | | | EP | 3294762 | A1 | |
| | | | | AU | 2016262494 | A1 | |
| | | | | KR | 10-2017-0138543 | A | |
| | | | | CN | 107614516 | A | |
| JP | 2014-522663 | A | 08 September 2014 | US | 2014/0162315 | A1 | |
| | | | | claims | | | |
| | | | | WO | 2013/017813 | A1 | |
| | | | | EP | 2739755 | A1 | |
| JP | 2007-89512 | A | 12 April 2007 | US | 2007/0072267 | A1 | |
| | | | | claims | | | |
| JP | 2018-522565 | A | 16 August 2018 | US | 2018/0223293 | A1 | |
| | | | | claims | | | |
| | | | | WO | 2017/021541 | A1 | |
| | | | | EP | 3332005 | A1 | |
| | | | | KR | 10-2018-0037237 | A | |
| | | | | CN | 108350447 | A | |
| JP | 2019-505194 | A | 28 February 2019 | US | 2021/0355173 | A1 | |
| | | | | claims | | | |
| | | | | WO | 2017/109082 | A1 | |
| | | | | EP | 3184642 | A1 | |
| | | | | KR | 10-2018-0088733 | A | |
| | | | | CN | 108603213 | A | |
| WO | 2023/219575 | A1 | 16 November 2023 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006089329 A
- WO 2017021541 A
- WO 2017109082 A
- WO 2008103041 A

**Non-patent literature cited in the description**

- **KATRIN VIIGAND et al.** Genome Mining of Non-Conventional Yeasts: Search and Analysis of MAL Clusters and Proteins. *Genes*, 2018, vol. 9, 354
- *J. Mol. Biol.*, 1990, vol. 215, 403-410
- *Genes*, 2018, vol. 9, 354